# EUROPEAN PATENT SPECIFICATION

(11) **EP 1 228 043 B1**
(45) Date of publication and mention of the grant of the patent: **12.01.2005**
(21) Application number: 00971423.9
(22) Date of filing: 02.11.2000
(51) Int. Cl.: C07D 217/22, C07D 401/12, C07D 239/94, C07D 471/04, A61K 31/437, A61K 31/47, A61K 31/505

(54) **ISOQUINOLINE AND QUINAZOLINE DEIVATIVES HAVING A COMBINED 5HT1A, 5HT1B AND 5HT1D RECEPTOR ACTIVITY**
ISOCHINOLIN UND CHINAZOLINDERIVATE MIT KOMBINIERTER 5HT1A, 5HT1B UND 5HT1D REZEPTOR AKTIVITÄT
DERIVES D'ISOQUINOLINE ET DE QUINAZOLINE DOTES D'UNE ACTIVITE COMBINEE DE RECEPTEUR 5HT1A, 5HT1B ET 5HT1D

(30) Priority: 05.11.1999 GB 9926304; 20.07.2000 GB 0017880
(43) Date of publication of application: 07.08.2002
(73) Proprietor: SmithKline Beecham plc, Brentford, Middlesex TW8 9GS (GB)
(72) Inventor: GASTER, Laramie Mary SmithKline Beecham Pharmaceu., Harlow Essex CM19 5AW (GB); HEIGHTMAN, Thomas Daniel SmithKline Beecham Phar., Harlow Essex CM19 5AW (GB); PILLEUX, Jean-pierre SmithKline Beecham Pharmaceu., Harlow Essex CM19 5AW (GB)
(74) Representative: Giddings, Peter John
(86) International application number: PCT/EP2000/010908
(87) International publication number: WO 2001/032626

(56) References cited:
- WO-A-00/06575
- WO-A-98/47868
- WO-A-98/50358
- WO-A-99/31086

## Description

The present invention relates to novel isoquinoline and quinazoline derivatives, processes for their preparation, pharmaceutical compositions containing them and to their use in the treatment of various disorders.

WO 98/50358, WO 98/50346, WO 98/47868, WO 98/47885, WO 98/50543 and WO 99/31086 all disclose a series of novel compounds which are claimed to possess combined 5-HT_{1A}, 5-HT_{1B} and 5-HT_{1D} receptor affinity and which are useful in the treatment of various CNS disorders. WO 97/36867 and WO 98/14433 both disclose a series of lactam derivatives that are claimed to be selective agonist or antagonists of one or both of 5-HT_{1A} and 5-HT_{1D} receptors.

A structurally distinct class of compounds have now been found that also exhibit combined 5-HT_{1A}, 5-HT_{1B} and 5-HT_{1D} receptor affinity. It is expected that such compounds will be useful for the treatment and prophylaxis of various disorders. In a first aspect, the present invention therefore provides a compound of formula (I) or a pharmaceutically acceptable salt thereof: 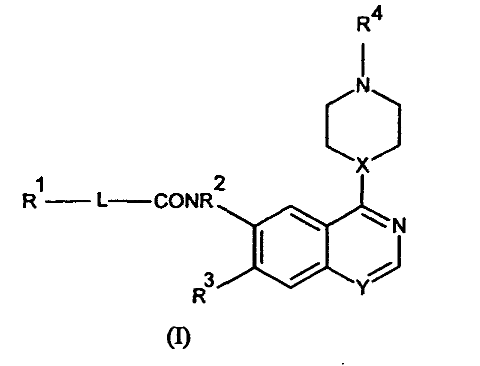 in which R¹ is selected from a group of formula (i) or (ii);
Group of formula (i) 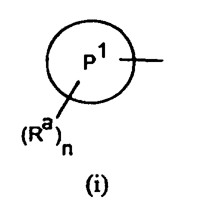 where P¹ is phenyl, naphthyl, a 5 or 6 membered heteroaryl ring containing 1 to 3 heteroatoms selected from oxygen, nitrogen and sulphur, a benzofused heterocyclic ring containing 1 to 3 heteroatoms selected from oxygen, nitrogen and sulphur, or a pyridofused heterocyclic ring containing 1 to 3 nitrogen atoms;
R^{a} is halogen, C₁₋₆alkyl, C₃₋₆cycloalkyl, CF₃, C₁₋₆alkoxy, OCF₃, hydroxy, cyano, nitro, hydroxyC₁₋₆alkyl, COC₁₋₆alkyl, CO₂R⁵, SO₂R⁵, NR⁵R⁶, CONR⁵R⁶, and SO₂NR⁵R⁶ where R⁵ and R⁶ are independently hydrogen or C₁₋₆alkyl;
n is 0, 1,2 or 3;
Group of formula (ii) 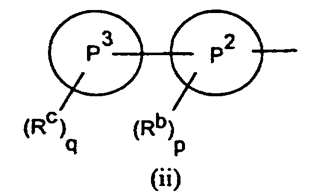 in which P² and p³ are independently as defined for P¹ above;
R^{b} and R^{c} are independently as defined for R^{a} above;
p and q are independently as defined for n above;
L is a single bond or NH;
R² is hydrogen or together with the group R³ forms a further group -CH = CH- or -(CR⁷R⁸)₂- where R⁷ and R⁸ are independently hydrogen or C₁₋₆alkyl;
R³ is hydrogen or together with R² forms a further group as defined above;
Y is N or CH;
X is N or CH;
R⁴ is hydrogen or C₁₋₆alkyl.

C₁₋₆alkyl groups whether alone or as part of another group may be straight chain or branched. Where used herein the term naphthyl is intended, unless otherwise stated, to denote both naphth-1-yl and naphth-2-yl groups. The term 'halogen' is used herein to describe, unless otherwise stated, a group selected from fluorine, chlorine, bromine or iodine.

### Within the definition of R¹ formula (i)

When P¹ is a 5 or 6 membered heteroaryl ring suitable examples include thienyl, furyl, pyrrolyl, triazolyl, imidazolyl, pyrazolyl, oxazolyl, thiazolyl, oxadiazolyl, isothiazolyl, isoxazolyl, thiadiazolyl, pyridyl, pyrimidyl and pyrazinyl. Where used herein the term "benzofused heterocyclic ring" is used to describe both 6,5 and 6,6 benzofused heteroaryl rings and benzofused non aryl heterocyclic rings. Suitable examples of benzofused heteroaryl rings include indolyl, benzofuryl, benzothienyl, quinolinyl and isoquinolinyl. Benzofused non aryl heterocyclic rings may be substituted by an oxo group. Suitable examples of benzofused non aryl heterocyclic rings include indolinyl, benzoxazinyl and benzoxazinonyl. When P¹ is pyridofused heterocyclic ring a preferred example is pyrazolopyridinyl. The rings defined for P¹ can be linked to the remainder of the molecule via any suitable carbon atom or, when present, a nitrogen atom.

Preferably P¹ is phenyl, naphthyl, quinolinyl, isoquinolinyl or a 5 or 6 membered heteroaryl ring such as pyridyl, oxazolyl, furyl, thiazolyl, imidazolyl, oxazolyl or pyrazolyl.

When n is not 0, R^{a} is preferably halogen (particularly fluorine, chlorine or bromine), a C₁₋₆alkyl group (particularly methyl, ethyl, isopropyl or t-butyl), CF₃, cyano, C₁₋₆alkoxy group (particularly methoxy or ethoxy) or SO₂R⁵ where R⁵ is methyl. When n is 2 or 3 the groups R^{a} may be the same or different. Preferably n is 1 or 2.

### Within the definition of R¹ formula (ii)

Suitable P² and P³ groups include those listed for P¹ above. The rings defined for P² and P³ can be linked to the remainder of the molecule via any suitable carbon atom or, when present, a nitrogen atom. Preferably P² is phenyl, naphthyl or a 5 or 6 membered heteroaryl ring such as thienyl, thiazolyl, oxazolyl and most preferably pyrazolyl. Preferably P³ is pyridyl or most preferably phenyl.

When p and/or q is not 0, R^{b} and R^{c} are preferably halogen (particularly fluorine, chlorine or bromine), a C₁₋₆alkyl group (particularly methyl, ethyl, isopropyl or t-butyl), CF₃, cyano, C₁₋₆alkoxy group (particularly methoxy or ethoxy). When p and/or q are 2 or 3 the groups R^{b} and R^{c} respectively can be the same or different. Preferably p is 0 or 1. Preferably q is 0, 1 or 2.

When L is NH, R¹ is preferably a group of formula (i). Preferably L is a single bond.

When R² together with a group R³ forms a group -(CR⁷R⁸)₂- both of the groups R⁷ and R⁸ are preferably hydrogen.

Preferably Y is CH.

Preferably X is N.

Preferably R⁴ is hydrogen or a methyl group.

Preferred compounds of this invention are examples E1 - E89 (as described below) or a pharmaceutically acceptable salt thereof Particularly preferred compounds according to this invention are:
2,3-Dihydro-1-(5-methyl-1-phenylpyrazol-4-yl carbonyl)-8-(4-methylpiperazin-1-yl)pyrrolo[3,2-g]isoquinoline,
1-(2-Fluorobenzoyl)-2,3-dihydro-8-(4-methylpiperazin-1-yl)pyrrolo[3,2-g]isoquinoline,
1-[1-(4-cyanophenyl)-5-ethylpyrazol-4-ylcarbonyl]-2,3-dihydro-8-(4-methylpiperazin-1-yl)pyrrolo[3,2-g]isoquinoline,
1-[1-(4-cyanophenyl)-5-methylpyrazol-4-ylcarbonyl]-2,3-dihydro-8-(4-methylpiperazin-1-yl)pyrrolo[3,2-g]isoquinoline,
2,3-Dihydro-1-(4-ethyl-2-methyloxazol-5-ylcarbonyl)-8-(4-methylpiperazin-1-yl)pyrrolo[3,2-g]isoquinoline
or a pharmaceutically acceptable salt thereof.

The compounds of formula (1) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of formula (I) may be prepared in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

In a further aspect the present invention provides a process for the preparation of a compound of formula (I) which comprises:
(a) when L is single bond, coupling a compound of formula (II):

   R¹―A (II)

   in which R¹ is as defined in formula (I) and A is an activated carboxylic acid group with a compound of formula (III); 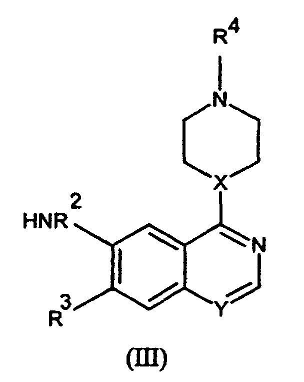 in which R², R³, R⁴, X and Y are as defined in formula (I); or
(b) where L is NH, coupling a compound of formula (IV)

   R¹―B (IV)

   in which R¹ is as defined in formula (I) and B is either -N=C=O or is NH₂ in the presence an appropriate urea forming agent, with a compound of formula (III) as defined above;
   and optionally thereafter for either process (a) or (b):
   - removing any protecting groups;
   - forming a pharmaceutically acceptable salt.

For process (a) suitable activated carboxylic acid groups include acyl chlorides or acyl bromides. Activated compounds of formula (II) can also be prepared by reaction of the corresponding carboxylic acid with a coupling agent such as carbonyldiimidazole dicyclohexylcarbodiimide, or diphenylphosphorylazide. Compounds of formulae (II) and (III) are typically reacted together in an inert solvent such as dichloromethane or dimethylformamide at ambient or elevated temperature in the presence of a base such as triethylamine or pyridine. Compounds of formula (I) may also be prepared by reaction of compounds of formula (II), where A is a carboxylic ester, with compounds of formula (III) in the presence of trimethylaluminium at ambient or elevated temperature in an inert solvent such as toluene.

For process (b) in which B is -N=C=O the reaction is conveniently effected in an organic solvent such as dichloromethane. For process (b) in which B is NH₂ suitable urea forming agents are carbonyl diimidazole, triphosgene and phosgene, and the reaction is carried out in an inert organic solvent such as dimethylformamide, tetrahydrofuran or dichloromethane at ambient or elevated temperature in the presence of a base such as triethylamine or pyridine.

It will be appreciated by those skilled in the art that it may be necessary to protect certain reactive substituents during some of the above procedures. Standard protection and deprotection techniques, such as those described in Greene T.W. 'Protective groups in organic synthesis', New York, Wiley (1981), can be used. For example, primary amines can be protected as phthalimide, benzyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures well known in the art.

Pharmaceutically acceptable salts may be prepared conventionally by reaction with the appropriate acid or acid derivative.

Intermediate compounds of formula (II), (III) and (IV) are commercially available, can be prepared using methods described herein or by analogous methods thereto or using standard procedures known in the art.

The involvement of serotonin receptors in a number of pharmacological effects has been reviewed by R. A. Glennon in "Serotonin Receptors: Clinical Implications", Neuroscience and Behavioural Reviews, 1990, 14, 35 and by L.O.Wilkinson and C.T. Dourish in "Serotonin Receptor Subtypes : Basic and Clinical Aspects" S. Peroutka Ed., John Wiley and Sons, New York, 1991 p.147.

Serotonin (5-hydroxytryptamine; 5-HT) receptors have been implicated in a number of pharmacological effects including mood disorders including depression, seasonal affective disorder and dysthymia, anxiety disorders, including generalised anxiety, panic disorder, agoraphobia, social phobia, obsessive compulsive disorder and post-traumatic stress disorder; memory disorders, including dementia, amnesic disorders and age-associated memory impairment; disorders of eating behaviours, including anorexia nervosa and bulimia nervosa, sleep disorders (including disturbances of Circadian rhythm), motor disorders such as Parkinson's disease, dementia in Parkinson's disease, neuroleptic-induced Parkinsonism and tardive dyskinesias, as well as other psychiatric disorders. Serotonin receptor ligands have been shown to be of use in the treatment of emesis and nausea and may also be of use in endocrine disorders such as hyperlactinaemia, vasospasm (particularly in the cerebral vasculature), cerebellar ataxia and hypertension, as well as disorders of the gastrointestinal tract where changes in motility and secretion are involved. They may also be of use in the treatment of sexual dysfunction and hypothermia.

Ligands with high affinity for the 5-HT₁ receptors are well recognised as having therapeutic utility for the treatment of the above conditions. For example: WO 95/31988 refers to the use of a 5-HT_{1D} receptor antagonist in conjunction with a 5-HT_{1A} receptor antagonist to treat CNS, endocrine and GI disorders; K. Rasmussen (Annual Reports in Medicinal Chemistry, (1995) 30, 1) describes the utility of 5-HT_{1A} receptor agonists and partial agonists in the treatment of various CNS disorders; P. Trouillas (Progress in Brain Research, C.I. de Zeeuw, P. Stara and J. Voogd, Eds. 1997, 144, 589) and G. Maura (J. Neurochemistry, 1996, 66, 202) propose that administration of agonist ligands selective for the 5-HT_{1A} receptor or for both 5-HT_{1A} and 5-HT_{1D} receptors should provide effective treatment for human cerebellar ataxias.

The present invention also provides for a compound of formula (I) or a pharmaceutically acceptable salt for use in the treatment of the aforementioned disorders. In particular, the invention provides for a compound of formula (I) or a pharmaceutically acceptable salt for use in the treatment or prophylaxis of depression.

In a further aspect the present invention provides for the use of a compound of formula (I) or a pharmaceutically acceptable salt thereof in the manufacture of a medicament for use in the treatment of disorders (particularly the aforementioned) in which a ligand with affinity for 5-HT_{1A}, 5-HT_{1B} and 5-HT_{1D} receptors is beneficial.

In a yet further aspect the invention provides a method of treating diseases or disorders (particularly the aforementioned disorders) in which a ligand with affinity for 5-HT_{1A}, 5-HT_{1B} and 5-HT_{1D} receptors is beneficial which comprises administering a safe and therapeutically effective amount to a patient in need thereof of compound of formula (I) or a pharmaceutically acceptable salt thereof.

It will be appreciated by those skilled in the art that the compounds according to the invention may advantageously be used in conjunction with one or more other therapeutic agents, for instance, a selective serotonin reuptake inhibitor (SSRI) antidepressant.

The affinities of the compounds of this invention for the 5-HT_{1A}, S-HT_{1B} and 5-HT_{1D} receptors can be determined by the following radioligand binding assay. HEK 293 cells expressing 5-HT_{1A} receptors (4 x 10⁷/ml) are homogenised in Tris buffer and stored in 1 in] aliquots. CHO cells expressing 5-HT_{1B} receptors (4 x 10⁷ cells/ml) are homogenised in Tris buffer and stored in 1.5 ml aliquots. CHO cells expressing 5-HT_{1D} receptors (0.563 x 10⁸/ml) are homogenised in Tris buffer and stored in 1 ml aliquots. 0.4 ml of a cell suspension is incubated with [³H]-5-HT (4 nM) for 5-HT_{1B/1D} receptors and [³H]-8-OH DPAT (1 nM) for 5-HT_{1A} receptors in Tris Mg HCl buffer (pH 7.7) and test drug, at 37°C for 45 minutes. Each test drug is tested at 10 concentrations (0.01 mM to 0.3 nM final concentration), with non-specific binding defined using 0.01 mM 5-HT. The total assay volume is 0.5 ml. Incubation is stopped by rapid filtration using a Packard Filtermate (filters pre-soaked in 0.3% polyethylenimine) and radioactivity measured by Topcount scintillation counting. pKi values are calculated from the IC₅₀ generated by an iterative least squares curve fitting programme.

All examples were tested in accordance with this radioligand binding assay and were found to have a pKi of greater than 7.5 at 5-HT_{1A}, 5-HT_{1B} and 5-HT_{1D} receptors.

The selectivity of the compounds of this invention for 5-HT_{1A}, 5-HT_{1B} and 5-HT_{1D} receptors can be determined using binding assay methods which are well known to those skilled in the art. All examples tested were found to have a greater than 20-fold selectivity over other binding sites within the CNS, in particular, other 5-HT receptor subtypes and dopaminergic receptors. Particularly preferred examples were found to have a greater than 100 fold selectivity over other binding sites.

The intrinsic activity of the compounds of this invention can be determined according to the following procedure. HEK293 cell membranes stably expressing human 5-HT_{1A} receptors and CHO cell membranes stably expressing human 5-HT_{1B} receptors are homogenised in HEPES/EDTA buffer and stored in 1 ml aliquots, and [³⁵S]GTPγS binding studies are carried out essentially as described by Lazareno *et al.,* (Life Sci., 1993, **52**, 449) with some minor modifications. Membranes from 10⁶ cells are preincubated at 30°C for 30 min in 20 mM HEPES buffer (pH 7.4) in the presence of MgCl₂ (3 mM), NaCl (100 mM), GDP (10 µM) and ascorbate (0.2 mM), with or without compounds. The reaction is started by the addition of 10 µl of [³⁵S]GTPγS (100 pM, assay concentration) followed by a further 30 minutes incubation at 30°C. Non-specific binding was determined using non-radiolabelled GTPγS (20 µM) added prior to the membranes. The reaction is terminated by rapid filtration through Whatman GF/B grade filters followed by 5 x 1 ml washes with ice cold HEPES (20 mM) /MgCl₂ (3 mM) buffer. Radioactivity is measured using liquid scintillation spectrometry. This procedure is hereafter referred to as the [³⁵S]GTPγS functional assay.

It has been found, using the [³⁵S]GTPγS functional assay, that certain compounds of formula (I) show varying levels of intrinsic efficacy, which is defined by a scale ranging from 1.0 to 0 (1 defines the maximum response elicited by the agonist 5-HT, 0 defines zero intrinsic efficacy). The difficulties in describing intrinsic activity of drugs acting at G protein coupled receptors is recognised in the art (Hoyer and Boddeke, Trends in Pharmacological Sciences, July 1993, [Vol. 14], page 270-275). Compounds of formula (I) which have low intrinsic activity in the [³⁵S]GTPγS functional assay are more likely to be antagonists *in vivo.* As disclosed in WO 95/31988, the simultaneous antagonism of pre-synaptic 5-HT_{1A/1B/1D} receptors will result in increased release of 5-HT *in vivo* and this should improve 5-HT neurotransmission. Accordingly, we believe that the compounds of this invention will be useful in the treatment of CNS disorders and that they will act as antidepressants *in vivo.*

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of compounds of the invention.

### Description 1

### 5-Nitro-1-chloroisoquinoline and 7-Nitro-1-chloroisoquinoline (D1)

A stirred solution of a mixture of 5- and 7-nitroisoquinolin-1-ols *(Chem. Pharm. Bull.* 1968, 16, 715; 3.8 g, 20 mmole) in POCl₃ (15 ml) under argon was heated under reflux for 3 h. After cooling the POCl₃ was removed *in vacuo* and the residue partitioned between DCM and saturated aqueous NaHCO₃. The organic layer was dried over MgSO₄, filtered and concentrated to dryness *in vacuo* giving the title compounds as a pale yellow solid (3.7 g, 89%). MS: m/z (MH⁺) = 209.

### Description 2

### 5-Nitro-1-(4-methylpiperazin-1-yl)isoquinoline and 7-Nitro-1-(4-methylpiperazin-1-yl)isoquinoline (D2)

A stirred solution of 1-chloro-5/7-nitroisoquinolines (D1, 1.8 g, 8.6 mmole) in DMF (20 ml) was treated with *N*-methyl piperazine (1.7 g, 17.2 mmole) and heated at 100° for 3 h. After cooling, the solvent was removed *in vacuo* and the residue partitioned between water and EtOAc. The organic layer was dried over MgSO₄, filtered and concentrated to dryness *in vacuo.* The residue was passed through a silica gel column eluting with DCM/MeOH/NH₄OH 19:1:0.1 giving the title compounds as a red oil (1.2 g, 51%).
MS: m/z (MH⁺) = 273.

### Description 3

### 7-Amino-1-(4-methylpiperazin-1-yl)isoquinoline (D3)

A stirred solution of 1-(4-methylpiperazin-1-yl)-5/7-nitroisoquinolines (D2, 1.2 g, 4.4 mmole) in EtOH (50 ml) was treated with cone. HCl (5 ml) and SnCl₂.2H₂O (4 g, 17.6 mmole) and heated under reflux for 15 h. The suspension was poured onto ice and basified with 40% NaOH. The aqueous suspension was extracted twice with EtOAc, and the combined organic layers washed with brine, dried over MgSO₄, concentrated *in vacuo* and purified by silica gel chromatography eluting with DCM/MeOH/NH₄OH 19:2:0.1 to give the title compound as a red-brown solid (140 mg, 13%).
MS: m/z (MH⁺) = 243. 1H-NMR (250 MHz, CDCl₃): δ (ppm): 7.96 (d, 1H), 7.59 (d, 1H), 7.22 (s, 1H), 7.14 (d, 1H), 7.07 (d, 1H), 3.98 (br. s, 2H), 3.38 (br. s, 4H), 2.69 (br. s, 4H), 2.40 (s, 3H).

### Description 4

### 4-(4-Methyl-piperazin-1-yl)-6-nitro-quinazoline (D4)

A stirred solution of 4-chloro-6-nitroquinazoline *(J. Chem. Soc.* 1948, 360; 1.0 g, 4.8 mmole) in dry DCM under Ar was treated with N-methylpiperazine (1.06 ml, 9.6 mmole). After 18 h the solution was evaporated to dryness *in vacuo* and the residue partitioned between DCM and water. The organic phase was dried over MgSO₄, filtered and concentrated to dryness *in vacuo* to give the title compound as an orange gum (1.18 g, 90%).
MS: m/z (MH⁺) = 274. 1H-NMR (250 MHz, CDCl₃): δ (ppm): 8.83 (d, 1H), 8.75 (s, 1H), 8.46 (dd, 1H), 7.94 (d, 1H), 4.00 (t, 4H), 2.65 (t, 4H), 2.41 (s, 3H).

### Description 5

### 6-Amino-4-(4-metyl-piperazin-1-yl)quinazoline (D5)

A stirred suspension of 4-(4-methylpiperazin-1-yl)-6-nitro-quinazoline (D4, 1.2 g, 4.3 mmole) and 10% Pd/C (150 mg) in EtOH was hydrogenated at 1 bar for 48 h, then filtered through kieselguhr. The filtrate and washings were concentrated to dryness to give the title compound as a yellow solid (1.01 g, 97%).
MS: m/z (MH⁺) = 244. 1H-NMR (250 MHz, CDCl₃): δ (ppm): 8.41 (s, 1H), 7.57 (d, 1H), 7.21 (d, 1H), 6.93 (s, 1H), 3.71 (br. s, 2H), 3.56 (br. s, 4H), 2.71 (br. s, 4H), 2.39 (s, 3H).

### Description 6

### Benzyl (1-benzyl)indole-6-carboxylate (D6)

A stirred solution of indole-6-carboxylic acid (5.4 g, 33 mmole) in DMF (100 ml) under argon was treated portionwise with sodium hydride (60% oil dispersion, 4.0 g, 100 mmole). After 30 minutes, benzyl bromide (16.5 ml, 140 mmole) was added and the solution stirred for 16 h at room temperature. After removal of the DMF *in vacuo* the residue was partitioned between water and EtOAc. The aqueous phase was extracted with EtOAc and the combined organics washed with brine, dried over Na₂SO₄ and concentrated *in vacuo.* The crude product was purified by flash chromatography, eluting with DCM/hexanes (40:60) to afford a white crystalline solid (13.2 g, 90%).
MS: m/z (MH⁺) = 342. 1H-NMR (250 MHz, CDCl₃): δ (ppm): 8.12 (s, 1H), 7.84 (dd, 1H), 7.66 (d, 1H), 7.40 (m, 10H), 7.12 (dd, 1H), 6.58 (d, 1H), 5.36 (s, 4H).

### Description 7

### Benzyl (1-benzyl)indoline-6-carboxylate (D7)

A stirred solution of benzyl (1-benzyl)indole-6-carboxylate (D6, 6.8 g, 20 mmole) in acetic acid (35 ml) was treated with sodium cyanoborohydride (3.8 g, 60 mmole) in two portions. After 16 h, the acetic acid was removed *in vacuo* and the residue diluted with water, then basified with 2 M aqueous NaOH solution. Extraction with EtOAc followed by washing with brine, drying over Na₂SO₄ and concentration *in vacuo* afforded a pale yellow solid (6.9 g, quant.).
MS: m/z (MH⁺) = 344. 1H-NMR (250 MHz, CDCl₃): δ (ppm): 7.30 (m, 11H), 7.18 (d, 1H), 7.11 (s, 1H), 5.32 (s, 2H), 4.29 (s, 2H), 3.35 (t, 2H), 3.00 (t, 2H).

### Description 8

### 1-Benzylindoline-6-carboxylic acid (D8)

A solution of benzyl (1-benzyl)indoline-6-carboxylate (D7, 7.55 g, 22 mmole) in 1,4-dioxane (200 ml) was treated with 1 M aq. NaOH (200 ml), and heated under reflux for 16 h. Volatiles were removed *in vacuo* and the residue diluted with water, then acidified with 5 M aqueous HCl. Extraction with EtOAc, followed by washing with brine, drying over Na₂SO₄ and concentration *in vacuo* afforded a yellow solid (5.4 g, 85%).
MS: m/z (MH⁺) = 342. 1H-NMR (250 MHz, *d*₆-DMSO) δ (ppm): 12.58 (s, 1H), 7.39-7.28 (m, 5H), 7.24 (dd, 1H), 7.11 (d, 1H), 7.02 (d, 1H), 4.31 (s, 2H), 3.35 (t, 2H), 2.96 (t, 2H).

### Description 9

### 1-Benzylindoline-6-carboxylic acid (2,2-dimethoxyethyl)amide (D9)

A stirred solution of 1-benzylindoline-6-carboxylic acid (D8, 5.4 g, 19 mmole) in DMF (300 ml) was treated with triethylamine (3.5 ml, 25 mmole), EDC (4.6 g, 24 mmole), HOBt.H₂O (3.7 g, 24 mmole) and aminoacetaldehyde dimethyl acetal (3.2 ml, 30 mmole). After 16h, the DMF was removed *in vacuo* and the residue diluted with water. Extraction with EtOAc, followed by washing with aqueous NaHCO₃, then brine, drying over Na₂SO₄ and concentration *in vacuo* afforded a buff waxy solid (6.3 g, 99%).
MS: m/z (MH⁺) = 341. 1H-NMR (250 MHz, CDCl₃) δ (ppm): 7.26 (m, 5H), 7.08 (d, 1H), 7.01 (d, 1H), 6.95 (d, 1H), 6.24 (t, 1H), 4.47 (t, 1H), 4.30 (s, 2H), 3.57 (t, 2H), 3.43 (s, 6H), 3.36 (t, 2H), 2.99 (t, 2H).

### Description 10

### 1-Benzyl-2,3-dihydro-8-hydroxypyrrolo[3,2-g]isoquinoline (D10)

A solution of I-benzylindoline-6-carboxylic acid (2,2-dimethoxyethyl)amide (D9, 2.4 g, 7 mmole) in anhydrous THF (100 ml) was treated with BF₃.Et₂O and the reaction mixture heated at 80°C for 16h under argon. The DMF was removed *in vacuo* and the residue partitioned between brine and EtOAc. Extraction of the aqueous with EtOAc, followed by washing of the combined organic phases with aqueous NaHCO₃, then brine, drying over Na₂SO₄ and concentration *in vacuo* afforded a brown solid (1.83 g, 94%).
MS: m/z (MH⁺) = 277. 1H NMR (250 MHz, CDCl₃): δ (ppm): 10.80 (s, 1H), 7.33 (m, 7H), 6.95 (d, 1H), 6.45 (d, 1H), 4.43 (s, 2H), 3.42 (t, 2H), 3.10 (t, 2H).

### Description 11

### 1-Benzyl-2,3-dihydro-8-trifluoromethanesulfonyloxy-pyrrolo[3,2-g]isoquinoline (D11)

An ice-cooled solution of 1-benzyl-2,3-dihydro-8-hydroxypyrrolo[3,2-g]isoquinoline (D10, 0.83 g, 3 mmole) in anhydrous DCM (10 ml) and pyridine (10 ml) was treated dropwise with trifluoromethanesulfonic anhydride (0.56 ml, 3.3 mmole). The reaction mixture was stirred under Ar at 0°C for 5 minutes, then at room temperature for 16h. After removal of the volatiles *in vacuo,* the residue was filtered through a short pad of silica eluting with DCM, giving a pale green solid (1.04g, 85%).
1H NMR (250 MHz, CDCl₃) δ (ppm): 7.87 (d, 1H), 7.37 (m, 7H), 6.68 (s, 1H), 4.46 (s, 2H), 3.60 (t, 2H), 3.20 (t, 2H).

### Description 12

### 1-Benzyl-2,3-dihydro-8-(4-methylpiperazin-1-yl)pyrrolo[3,2-g]isoquinoline (D12)

A stirred solution of 1-benzyl-2,3-dihydro-8-trifluoromethanesulfonyloxy-pyrrolo[3,2-g]isoquinoline (D11, 680 mg, 1.67 mmole) in N-methylpiperazine (20 ml) was heated at 70° for 3 h, then concentrated to dryness *in vacuo.* Filtration through silica eluting with DCM/MeOH 19:1 gave the product as a brown solid (560 mg, 94%).
MS: m/z (MH⁺): 359. 1H NMR (250 MHz, CDCl₃,) δ (ppm): 7.91 (d, 1H), 7.33 (m, 6H), 7.08 (d, 1H), 6.70 (s, 1H), 4.41 (s, 2H), 3.55 (t, 2H), 3.27 (br. s, 4H), 3.16 (t, 2H), 2.55 (br. s, 4H), 2.37 (s, 3H).

### Description 13

### 2,3-Dihydro-8-(4-methylpiperazin-1-yl)pyrrolo[3,2-g]isoquinoline (D13)

A stirred solution of 1-benzyl-2,3-dihydro-8-(4-methylpiperazin-1-yl)pyrrolo[3,2-g]isoquinoline (D12, 250 mg, 0.7 mmole) in EtOH (50 ml) under argon was treated with ammonium formate (1.5 g, 2.4 mmole) and 10% Pd/C (150 mg) and the reaction mixture refluxed for 20h under Ar atmosphere. The catalyst was removed by filtration through Kieselguhr and the filtrate concentrated *in vacuo.* The residue was purified by flash chromatography affording a brown solid (50 mg, 27%).
MS: m/z (MH⁺) = 269. 1H NMR (250 MHz, CDCl₃): δ (ppm): 7.93 (d, 1H), 7.44 (s, 1H), 7.11 (d, 1H), 7.05 (s, 1H), 3.70 (t, 2H), 3.38 (br. s, 4H), 3.18 (t, 2H), 2.72 (br. s, 4H), 2.42 (s, 3H).

### Description 14

### Ethyl (N,N-dimethylamino)methylene acetoacetate (D14)

A solution of ethyl acetoacetate (2.6g, 20 mmole) and N,N-dimethylformamide dimethylacetal (6.6 ml, 50 mmole) was refluxed for 1.5h. After concentration *in vacuo,* the residue was diluted with brine, extracted with EtOAc. The combined organic phases were washed with brine, dried over Na₂SO₄ and concentrated *in vacuo* affording an orange oil (3.4g, 91 %).
1H-NMR (250 MHz, CDCl₃): δ (ppm): 7.67 (s, 1H), 4.22 (q, 2H), 2.95 (br. s, 6H), 2.32 (s, 3H), 1.30 (t, 3H).

### Description 15

### Ethyl 1-(4-cyanophenyl)-5-methylpyrazole-4-carboxylate (D15)

A solution of ethyl (N,N-dimethylamino)methylene acetoacetate (D14, 0.37g, 2.0 mmole), 4-cyanophenyl hydrazine hydrochloride (0.34 g, 2.0 mmole) and triethylamine (0.31 ml, 2.2 mmole) in EtOH (10 ml) was stirred at room temperature for 2h. The ethanol was removed *in vacuo* and the residue partitioned between H₂O and Et₂O. The aqueous phase was extracted with ether and the combined organic phases washed with 1M aqueous HCl, followed by brine, then dried over Na₂SO₄ and concentrated *in vacuo,* affording a yellow solid (0.45 g, 88%).
MS: m/z (MH⁺) = 256. 1H-NMR (250 MHz, CDCl₃): δ (ppm): 8.06 (s, 1 H), 7.80 (d, 2H), 7.61 (d, 2H), 4.35 (q, 2H), 2.64 (s, 3H), 1.41 (t, 3H).

### Description 16

### Ethyl 1-(4-cyanophenyl)-5-ethylpyrazole-4-carboxylate (D16)

The title compound was prepared from ethyl (N,N-dimethylamino)methylene propionoacetate (prepared from ethyl propionoacetate as per D14, 1.17 g, 5.9 mmole) and 4-cyanophenyl hydrazine hydrochloride (1.0 g, 5.9 mmole) according to the procedure for Description 15: pale brown crystals (1.4 g, 88%).
MS: m/z (MH⁺) = 270. 1H-NMR (250 MHz, CDCl₃): δ (ppm): 8.06 (s, 1H), 7.83 (d, 2H), 7.59 (d, 2H), 4.34 (q, 2H), 3.02 (q, 2H), 1.39 (t, 3H), 1.23 (t, 3H).

### Description 17

### 1-Benzyl-2,3-dihydro-8-(4-tert-butyloxycarbonylpiperazin-1-yl)pyrrolo(3,2- g]isoquinoline (D17)

A solution of 1-benzyl-2,3-dihydro-8-trifluoromethanesulfonyloxy-pyrrolo[3,2-g]isoquinoline (D11 , 408 mg, 1.0 mmole) and N-tert-butyloxycarbonyl piperazine (3.7g, 20 mmole) in DMF (4ml) was heated at 70°C for 5h. After concentration *in vacuo,* the residue was purified by flash chromatography on a silica column eluting with pentane/EtOAc (100:0 to 80:20) to afford an orange solid (270 mg, 61%).
MS: m/z (MH⁺): 445. 1H-NMR (250 MHz, CDCl₃, free base): δ (ppm): 7.91 (d, 1H), 7.36 (m, 6H), 7.11 (d, 1H), 6.66 (s, 1H), 4.42 (s, 2H), 3.59 (t, 2H), 3.48 (m, 4H), 3.16 (m, 6H), 1.48 (s, 9H).

### Description 18

### 1-Benzyl-2,3-dihydro-8-(piperazin-1-yl)pyrrolo[3,2-g]isoquinoline (D18)

A solution of 1-benzyl-2,3-dihydro-8-(4-*tert*-butyloxycarbonylpiperazin-1-yl)pyrrolo[3,2-g]isoquinoline (D17, 260 mg, 0.58 mmole) in TFA (1 ml) was stirred at room temperature for 1h. The TFA was removed *in vacuo* and the residue dissolved in DCM, and washed with an aqueous saturated solution of K₂CO₃. The organic phase was dried over Na₂SO₄, then concentrated *in vacuo* affording an orange solid (160 mg, 80%).
MS: m/z (MH⁺): 345. 1H NMR (250 MHz, CDCl₃, free base): δ (ppm): 7.92 (d, 1H), 7.33 (m, 6H), 7.09 (d, 1H), 6.70 (s, 1H), 4.42 (s, 2H), 3.58 (t, 2H), 3.17 (m, 6H), 2.97 (m, 4H).

### Description 19

### 1-Benzyl-2,3-dihydro-8-(4-trifluoroacetylpiperazin-1-yl)pyrrolo[3,2-g]isoquinoline (D19)

A solution of 1-benzyl-2,3-dihydro-8-(piperazin-1-yl)pyrrolo[3,2-g]isoquinoline (D18, 0.64 g, 1.85 mmole) in THF (10 ml) was treated with 2-trifluoroacetoxypyridine (0.39 g, 2.05 mmole) and the reaction mixture stirred at room temperature for 1.5h. The THF was removed *in vacuo,* and the residue diluted with DCM, washed with brine, dried over Na₂SO₄ and concentrated *in vacuo.* Flash chromatography using EtOAc/pentane (15:85) afforded a yellow solid (0.5 1g, 63%).
MS: m/z (MH⁺) = 441. 1H-NMR (250 MHz, CDCl₃, free base): δ (ppm): 7.91 (d, 1H), 7.38 (m, 6H), 7.15 (d, 1H), 6.53 (s, 1H), 4.43 (s, 2H), 3.65 (m, 6H), 3.21 (m, 4H).

### Description 20

### 1-Formyl-2,3-dihydro-8-(4-trifluoroacetylpiperazin-1-yl)pyrrolo[3,2-g]isoquinoline (D20)

A solution of 1-benzyl-2,3-dihydro-8-(4-trifluoroacetylpiperazin-1-yl)pyrrolo[3,2-g]isoquinoline (D19, 0.51g, 1.15 mmole) and 10% Pd/C (0.5g) in formic acid (60 ml) was stirred at 80°C for 1.5h. After cooling, the reaction mixture was filtered through Kieselguhr and concentrated *in vacuo.* Flash chromatography using EtOAc/Pentane (90:10) afforded a pale yellow solid (220mg, 5%).
MS: m/z (MH⁺) = 379. 1H NMR (250 MHz, CDCl₃, free base, two rotamers): δ (ppm): 9.14 (s, 0.5H), 8.70 (s, 0.5H), 8.65 (s, 0.5H), 8.10 (m, 1H), 7.72 (s, 0.5H), 7.65 (s, 0.5H), 7.60 (s, 0.5H), 7.23 (m, 1H), 4.25 (t, 1H), 4.17 (t, 1H), 3.94 (m, 4H), 3.46 (m, 4H).

### Description 21

### 2,3-Dibydro-8-(4-trifluoroacetylpiperazin-1-yl)pyrrolo[3,2-g]isoquinoline (D21)

A solution of 1-formyl-2,3-dihydro-8-(4-trifluoroacetylpiperazin-1-yl)pyrrolo[3,2-g]isoquinoline (D20, 210 mg, 0.55 mmole) in 2 M aqueous HCl (10 ml) was stirred at 70°C for 20 minutes. After cooling to 0°C, the reaction mixture was neutralized with solid K₂CO₃, then extracted with CHCl₃. The combined organic extracts were dried over Na₂SO₄, then concentrated *in vacuo* affording a pale brown solid (190 mg, 98%).
MS: m/z (MH⁺) = 351. 1H NMR (250 MHz, CDCl₃, free base): δ (ppm): 7.93 (d, 1H), 7.47 (s, 1H), 7.18 (d, 1H), 7.01 (s, 1H), 3.92 (br. t, 2H), 3.85 (br. t, 2H), 3.69 (t, 2H), 3.38 (m, 4H), 3.20 (t, 2H).

### Description 22

### Ethyl 4-ethyl-2-methyloxazole-5-carboxylate (D22)

A stirred solution of ethyl 2-chloropropionylacetate (12.4 g, 69 mmole) in glacial AcOH (24 ml) was treated with acetamide (8.2 g, 139 mmole) and heated at reflux, under argon, for 18h. On cooling, the mixture was concentrated *in vacuo,* diluted with H₂O (50 ml) and extracted with Et₂O (2 x 150 ml). The combined organic extracts were washed with dilute aqueous K₂CO₃ solution, dried (MgSO₄) and concentrated to dryness *in vacuo.* The residue was purified by silica gel chromatography, eluting with Et₂O/petrol gradient, to afford the title compound as a yellow oil (3.64 g, 29%).
MS: m/z (MH⁺) = 184. ¹H NMR (250 MHz, CDCl₃): δ (ppm): 4.36 (q, 2H), 2.85 (q, 2H), 2.50 (s, 3H), 1.39 (t, 3H), 1.24 (t, 3H).

### Description 23

### tert-Butyl 1,2,3,6-tetrahydro-4-trimethylstannyl-pyridine-1-carboxylate (D23)

A suspension of tert-butyl 1,2,3,6-tetrahydro-4-[(trifluommethyl)sulfonyloxy)-pyridine-1-carboxylate *(Synthesis* 1991, 993; 500 mg, 1.51 mmole), hexamethylditin (550 mg, 1.68 mmole) and LiCl (374 mg, 9.01 mmole) in THF (20 ml) was degassed with Ar, treated with Pd(PPh₃)₄ (175 mg, 0.15 mmole) and heated under reflux for 16 h. After cooling the solution was diluted with petrol and washed with saturated aqueous NaHCO₃, dried (Na₂CO₃) and concentrated to dryness. The residue was filtered through silica (EtOAc/petrol 1:9) to give a colourless oil (506 mg, 97%).
¹H NMR (250 MHz, CDCl₃,): δ (ppm): 5.77 (br. s, 1H), 3.91 (m, 2H), 3.47 (t, 2H), 2.28 (br. s, 2H), 1.47 (s, 9H), 0.12 (s, 9H).

### Description 24

### 1-Benzyl-2,3-dibydro-8-(1-tert-butyloxycarbonyl-1,2,3,6-tetrabydropyridin-4-yl)pyrrolo[3,2-g]isoquinoline (D24)

A solution of 1-benzyl-2,3-dihydro-8-trifluoromethanesulfonyloxy-pyrrolo[3,2-g]isoquinoline (D11, 1.90 g, 4.66 mmole) and tert-butyl 1,2,3,6-tetrahydro-4-trimethylstannylpyridine-1-carboxylate (D23, 2.13 g, 6.16 mmole) in DMF (20ml) was degassed with Ar for 20 min, treated with CuI (27 mg, 0.14 mmole) and Pd(PPh₃)₂Cl₂ (101 mg, 0.14 mmole), and heated at 110° for 16 h. The mixture was concentrated to dryness *in vacuo* and partitioned between saturated NaHCO₃ and DCM. The organic layer was dried (Na₂SO₄) and concentrated to dryness. Filtration of the residue (silica, EtOAc/petrol 1:19) gave the product as a yellow oil (853 mg, 42%).
MS: m/z (MH⁺) = 442. ¹H NMR (250 MHz, CDCl₃): δ (ppm): 8.19 (d, 1H), 7.43 (s, 1H), 7.37-7.26 (m, 6H), 6.70 (br. s, 1H), 5.80 (br. s, 1H), 4.39 (s, 2H), 4.04 (br. s, 2H), 3.63 (t, 2H), 3.57 (t, 2H), 3.18 (t, 2H), 2.54 (br. s, 2H), 1.51 (s, 9H).

### Description 25

### 1-Benzyl-2,3-dihydro-8-(1-tert-butyloxycarbonylpiperidin-4-yl)pyrrolo[3,2-g]isoquinoline (D25)

A solution of 1-benzyl-2,3-dihydro-8-(1-*tert*-butyloxycarbonyl-1,2,3,6-tetrahydropyridin-4-yl)pyrrolo[3,2-g]isoquinoline (D24, 850 mg, 1.93 mmole) in MeOH (30 ml) was hydrogenated over Pd/C (10%, 1.2 g) for 18 h. After filtration through Kieselguhr the solvent was removed *in vacuo* giving a colourless glass (842 mg, 99%).
MS: m/z (MH⁺) = 444.

### Description 26

### 1-Benzyl-2,3-dihydro-8-(piperidin-4-yl)pyrrolo[3,2-g]isoquinoline (D26)

A solution of 1-benzyl-2,3-dihydro-8-(1-*tert*-butyloxycarbonylpiperidin-4-yl)pyrrolo[3,2-g]isoquinoline (D25, 840 mg, 1.90 mmole) in trifluoroacetic acid (10 ml) was stirred at room temperature for 15 h, then concentrated to dryness *in vacuo.* The residue was partitioned between saturated aqueous K₂CO₃ and DCM, and the aqueous layer extracted twice with DCM. Combined organic layers were dried (Na₂SO₄) and concentrated to dryness giving a yellow oil (350 mg, 54%). MS: m/z (MH⁺) = 344.

### Description 27

**1-Benzyl-2,3-dihydro-8-(1-methylpiperidin-4-yl)pyrrolo[3,2-g]isoquinoline (D27)** A solution of 1-benzyl-2,3-dihydro-8-(piperidin-4-yl)pyrrolo(3,2-g]isoquinoline (D26, 350 mg, 1.02 mmole) in methanol (10 ml) was treated with formaldehyde (37% aqueous, 0.60 ml) and NaBH₄CN (200 mg, 2.86 mmole), and stirred at room temperature for 2 h. The solution was concentrated *in vacuo* and the residue partitioned between DCM and 1 M NaOH solution. The aqueous layer was extracted with DCM and the combined organic layers dried (Na₂SO₄) and concentrated to dryness *in vacuo,* giving a yellow oil (190 mg, 52%). MS: m/z (MH⁺) = 358.

### Description 28

### 1-Formyl-2,3-dihydro-8-(1-methylpiperidin-4-yl)pyrrolo[3,2-g]isoquinoline (D28)

The title compound was prepared from 1-benzyl-2,3-dihydro-8-(1-methylpiperidin-4-yl)pyrrolo[3,2-g]isoquinoline (D27, 190 mg, 0.53 mmole) according to the procedure in Description D20: pale brown crystals (80 mg, 63%). MS: m/z (MH⁺) = 296.

### Description 29

### 2,3-Dihydro-8-(1-methylpiperidin-4-yl)pyrrolo[3,2-g]isoquinoline (D29)

The title compound was prepared from 1-formyl-2,3-dihydro-8-(1-methylpiperidin-4-yl)pyrrolo[3,2-g]isoquinoline (D28, 80 mg, 0.27 mmole) according to the procedure in Description D21: buff crystals (68 mg, 94%).
MS: m/z (MH⁺) = 268. ¹H NMR (250 MHz, CDCl₃): δ (ppm): 8.22 (d, 1H), 7.49 (s, 1 H), 7.33 (d, 1H), 6.96 (s, 1H), 4.27 (br. s, 1H), 3.69 (t, 2H), 3.48 (m, 2H), 3.21 (t, 2H), 2.83 (s, 3H), 2.80 (m, 2H), 2.66 (m, 2H), 1.93 (br. d, 2H).

### Description 30

### Ethyl 1-(3-cyanophenyl)-5-trifluoromethylpyrazole-4-carboxylate (D30)

A solution of ethyl ethoxymethylene-3-oxo-4,4,4-trifluorobutyrate (1.7 g, 6 mmol) in EtOH (4ml) was added over a 1.5h period to a solution of 3-cyanophenylhydrazine (1.0 g, 6 mmol) and triethylamine (0.8 ml, 6.6 mmol) in EtOH (20 ml) at 0°C. The reaction was allowed to warm up to room temperature and stirred for 20h. After removal of the EtOH *in vacuo,* the residue was purified by silica chromatography affording a yellow solid (180 mg, 10%).
1H NMR (400 MHz, CDCl₃): δ (ppm): 8.15 (s, 1H), 7.78 (dd, 1H), 7.68 (s, 1H), 7.65 (s, 1H), 7.63 (d, 1H), 4.38 (q, 2H), 1.39 (t, 3H).

### Description 31

### Ethyl 1-(4-cyanophenyl)-3-trifluoromethylpyrazole-4-carboxylate (D31)

A solution of ethyl 3-trifluoromethylpyrazole-4-carboxylate (8.5 g, 41 mmol) in DMF (200 ml) was treated portionwise with sodium hydride (60% oil dispersion, 1.95 g, 48 mmol) and the reaction mixture stirred at room temperature under Ar for I h. 4-fluorobenzonitrile (5.3 g, 44 mmol) was added and the mixture stirred at 110°C for 16h. After cooling to room temperature, the reaction was quenched with water, then concentrated *in vacuo.* The residue was purified by silica chromatography affording a white solid (11.1g, 88%).
1H NMR (400 MHz, CDCl₃): δ (ppm): 8.56 (s, 1H), 7.90 (d, 2H), 7.84 (d, 2H), 4.37 (q, 2H), 1.39 (t, 3H).

### Description 32

### 3-Aminosalicylic acid ethyl ester (D32)

A solution of 3-amino salicylic acid (0.75g, 4.9 mmol) in EtOH (30 ml) and concentrated H₂SO₄ (5 ml) was stirred at room temperature for 3h. After concentration *in vacuo,* the residue was diluted with water, treated with solid K₂CO₃ and extracted with EtOAc.

After drying over Na₂SO₄, concentration of the organic phase *in vacuo* afforded a brown oil 0.41 g (46%).
MS: m/z (MH⁺) =182. 1H-NMR (400 MHz, CDCl₃): δ (ppm): 7.25 (d, 1H), 6.88 (d, 1H), 6.71 (t, 1H), 4.37 (q, 2H), 1.41 (t, 3H).

### Description 33

### 4H-3-Oxo-benz[1,4]oxazine-8-carboxylic acid ethyl ester (D33)

A solution of 3-aminosalicylic acid ethyl ester (D32) (0.41 g, 2.1 mmol) in DMF (20 ml) was treated with chloroacetyl chloride (0.185 ml, 2.3 mmol) and stirred at room temperature for 20 minutes. Solid K₂CO₃ (1.38 g, 10 mmol) was then added portionwise and the reaction stirred at room temperature for 16 h under Ar. After concentration *in vacuo,* the residue was partitioned between H₂O and CH₂Cl₂. The organic phase was washed with brine, dried over Na₂SO4, then concentrated *in vacuo,* affording a brown solid (0.41g, 88%).
MS: m/z (MH⁺) = 220. 1H-NMR (250 MHz, CDCl₃): δ (ppm): 8.72 (s, 1H), 7.52 (dd, 1H), 7.02 (d, 1H), 6.99 (s, 1H), 4.72 (s, 2H), 4.36 (q, 2H), 1.36 (t, 3H).

### Description 34

### Ethyl 1-(4-methoxyphenyl)-5-methylpyrazole-4-carboxylate (D34)

The title compound was prepared from ethyl (N,N-dimethylamino)methylene acetoacetate (D14, 1.02 g, 5.1 mmole) and 4-methoxyphenyl hydrazine hydrochloride (0.89 g, 5.1 mmole) according to the procedure for Description 15: pale brown crystals (0.99 g, 84%). MS: m/z (MH⁺) = 233.

### Description 35

### Ethyl 1-(4-methoxyphenyl)-5-ethylpyrazole-4-carboxylate (D35)

The title compound was prepared from ethyl (N,N-dimethylamino)methylene propionoacetate (prepared from ethyl propionoacetate as per D14, 1.08 g, 5.4 mmole) and 4-methoxyphenyl hydrazine hydrochloride (0.92 g, 5.4 mmole) according to the procedure for Description 15: buff solid (1.21 g, 91%). MS: m/z (MH⁺) = 289.

### Description 36

### Ethyl 1-(4-methoxyphenyl)-5-isopropylpyrazole-4-carboxylate (D36)

The title compound was prepared from ethyl (N,N-dimethylamino)methylene propionoacetate (prepared from ethyl isobutyrylacetate as per D14, 1.28 g, 6.2 mmole) and 4-methoxyphenyl hydrazine hydrochloride (1.08 g, 6.2 mmole) according to the procedure for Description 15: pale yellow solid (1.31 g, 81%). MS: m/z (MH⁺) = 261.

### Description 37

### Ethyl 1-(4-cyanophenyl)-5-isopropylpyrazole-4-carboxylate (D37)

The title compound was prepared from ethyl (*N,N*-dimethylamino)methylene isobutyrylacetate (prepared from ethyl isobutyrylacetate as per D14, 1.70 g, 8.0 mmole) and 4-cyanophenyl hydrazine hydrochloride (1.36 g, 8.0 mmole) according to the procedure for Description 15: orange solid (2.1 g, 93%). MS: m/z (MH⁺) = 284.

### Description 38

### Ethyl 4-methyl-2-(4-cyanopbenyl)oxazole-5-carboxylate (D38)

A stirred solution of ethyl 2-chloroacetoacetate (5.0 g, 30 mmole) in glacial AcOH (10 ml) was treated with 4-cyanobenzamide *(J. Med. Chem.* 1991, 34, 1630; 8.9 g, 61 mmole) and heated at reflux, under argon, for 20h. On cooling, the mixture was concentrated *in vacuo,* diluted with H₂O (50 ml) and extracted with Et₂O (2 x 150 ml). The combined organic extracts were washed with dilute aqueous K₂CO₃ solution, dried (MgSO₄) and concentrated to dryness *in vacuo.* The residue was purified by silica gel chromatography, eluting with Et₂O/petrol gradient, to afford the title compound as a yellow oil (0.26 g, 3%). MS: m/z (MH⁺) = 257.

### Example 1

### 7-(4-Chlorobenzamido)-1-(4-methylpiperazin-1-yl)isoquinoline (E1)

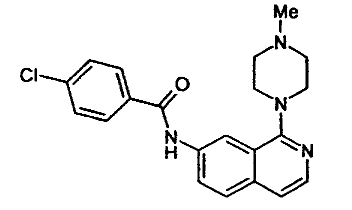

A stirred solution of 7-amino-1-(4-methylpiperazin-1-yl)isoquinoline (D3, 110 mg, 0.45 mmole) in DCM (5 ml) was treated with Et₃N (91 mg, 0.90 mmole), DMAP (cat.) and 4-chlorobenzoyl chloride (95 mg, 0.54 mmole). After 48 h the solution was diluted with DCM, washed with water, dried over MgSO₄, filtered and concentrated to dryness *in vacuo.* The residue was purified by flash chromatography giving the title compound as a pale brown solid (80 mg, 46%).
MS: m/z (MH⁺) = 381/383. 1H NMR (250 MHz, CDCl₃): δ (ppm): 8.59 (s, 1H), 8.15-8.05 (m, 2H), 7.90-7.80 (m, 2H), 7.80-7.60 (m, 2H), 7.55-7.45 (m, 2H), 7.23 (br. s, 1H), 3.49 (br. s, 4H), 2.78 (br. s, 4H), 2.43 (s, 3H).

### Example 2

### 7-(4-Ethoxybenzamido)-1-(4-methylpiperazin-1-yl)isoquinoline (E2)

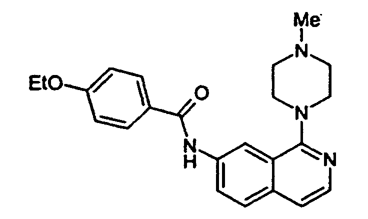

A stirred solution of 4-ethoxybenzoic acid (32 mg, 0.21 mmole), HOBt (29 mg, 0.21 mmole) and 7-amino-1-(4-methylpiperazin-1-yl)isoquinoline (D3, 35 mg, 0.14 mmole) in DMF (2 ml) was treated with polystyrylmethyl cyclohexyl carbodiimide (200 mg resin, 0.28 mmole) and heated at 60° for 72 h. After filtration the solution was concentrated to dryness and purified by preparative thin layer chromatography eluting with DCM/MeOH/NH₄OH 19:1:0.1 to give the title compound as an off white solid (30 mg, 53%).
MS: m/z (MH⁺) = 391. 1H NMR (250 MHz, CDCl₃): δ (ppm): 8.57 (s, 1H), 8.10 (d, 1H), 8.08 (s, 1H), 7.88 (d, 2H), 7.71 (s, 2H), 7.20 (d, 1H), 6.95 (d, 2H), 4.09 (q, 2H), 3.50 (br. s, 4H), 2.79 (br. s, 4H), 2.43 (s, 3H), 1.45 (t, 3H).

### Example 3

### 7-(3,4-Dichlorobenzamido-1-(4-methylpiperazin-1-yl)isoquinoline (E3)

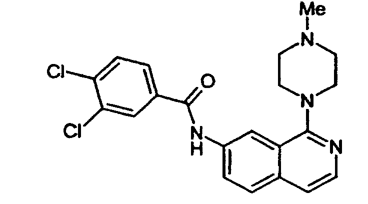

The title compound was prepared from 3,4-dichlorobenzoic acid and 7-amino-1-(4-methylpiperazin-1-yl)isoquinoline (D3, 35 mg, 0.14 mmole) according to the procedure for Example 2, giving a red-brown solid (46 mg, 79%).
MS: m/z (MH⁺) = 415. 1H NMR (250 MHz, CDCl₃): δ (ppm): 8.57 (s, 1H), 8.36 (br. s, 1H), 8.11 (d, 1H), 8.01 (d, 1H), 7.75-7.65 (m, 3H), 7.54 (d, 1H), 7.20 (d, 1H), 3.46 (br. s, 4H), 2.71 (br. s, 4H), 2.37 (s, 3H).

Examples E4 - E7 were prepared using a similar procedure to that used for Example 2.

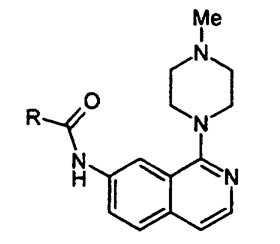

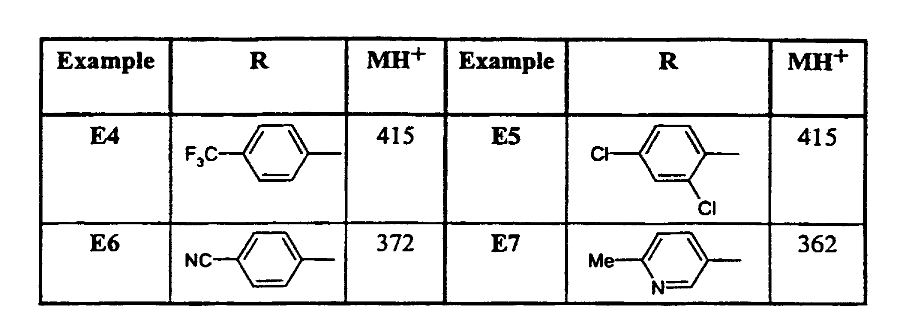

### Example 8

### 6-(2-Cblorobeozamido)-4-(4-metbylpiperazin-1-yl)quinazoline (E8)

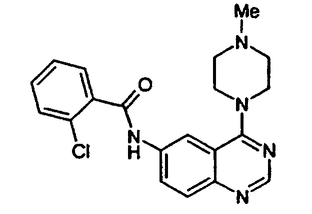

The title compound was prepared from 2-chlorobenzoic acid and 6-amino-4-(4-methylpiperazin-1-yl)quinazoline (D5, 97 mg, 0.39 mmole) according to the procedure for Example 2, giving a brown glass (115 mg, 74%).
MS: m/z (MH⁺) = 382. 1H NMR (250 MHz, CDCl₃): δ (ppm): 8.78 (s, 1H), 8.67 (s, 1H), 8.52 (br. s, 1H), 7.85 (d, 1H), 7.75 (dd, 1H), 7.56 (dd, 1H), 7.43 (m, 3H), 3.87 (br. s, 4H), 2.69 (br. s, 4H), 2.39 (s, 3H).

**Examples E9 - E12** were prepared using a similar procedure to that described for Example 8.

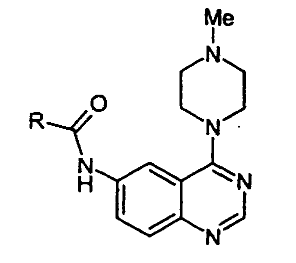

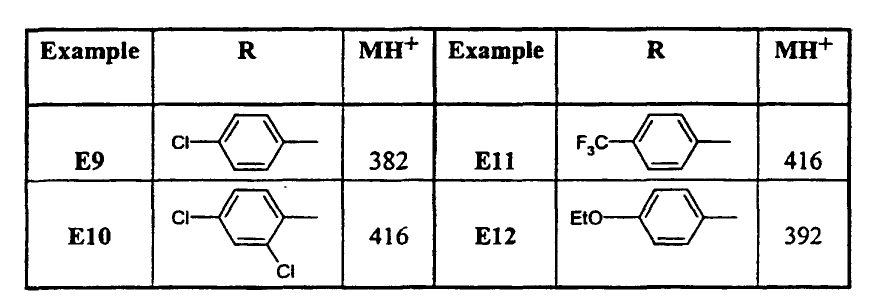

### Example 13

### 1-(4-Chlorobenzoyl)-2,3-dihydro-8-(4-methylpiperazin-1-yl)pyrrolo[3,2-g]isoqninoline (E13)

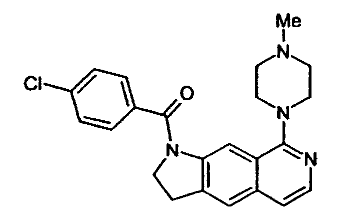

A stirred solution of 4-chlorobenzoic acid (44 mg, 0.28 mmole), *N,N*-diisopropyl carbodiimide(0.045 ml, 0.28 mmole) and HOBt.H₂O (43 mg, 0.28 mmole) in DMF (3 ml) was treated with a solution of 2,3-dihydro-8-(4-methylpiperazin-1-yl)pyrrolo[3,2-g]isoquinoline (D13, 50 mg, 0.18 mmole) and Et₃N (0.1 ml) in DMF (5 ml) and heated at 60°C for 16h under argon. After cooling, the solution was transferred to an SCX column, washing with MeOH followed by DCM. The product was eluted with 1M methanolic ammonia. Concentration *in vacuo* afforded a pale beige solid which was transformed into the HCl salt by treatment with 1 M HCl in Et₂O: yellow solid (62 mg, 78%).
MS: m/z (MH⁺) = 407. 1H NMR (250 MHz, CDCl₃, free base): δ (ppm): 8.90 (br. s, 1H), 8.07 (d, 1H), 7.61 (s, 1H), 7.57 (s, 2H), 7.48 (s, 1H), 7.45 (s, 1H), 7.15 (d, 1H), 4.18 (br. s, 2H), 3.38 (br. s, 4H), 3.28 (t, 2H), 2.70 (br. s, 4H), 2.38 (s, 3H).

### Example 14

### 1-(Quinolin-5-carbonyl)-2,3-dihydro-8-(4-methylpiperazin-1-yl)pyrrolo[3,2-g]isoquinoline (E14)

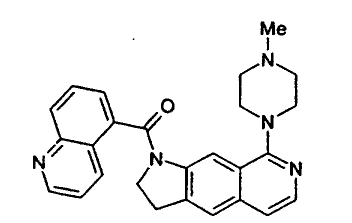

A stirred solution of 2,3-dihydro-8-(4-methylpiperazin-1-yl)pyrrolo[3,2-g]isoquinoline (D13, 75 mg, 0.28 mmole) and ethyl quinoline-5-carboxylate (84 mg, 0.42 mmole) in dry toluene (5 ml) was treated with AlMe₃ (2M in toluene, 0.56 ml) and heated under reflux for 18 h. The solution was passed directly through a silica column eluting with DCM/MeOH 9:1 to give the product which was converted to the hydrochloride using 1M HCl in Et₂O: yellow solid (78 mg, 66%).
MS: m/z (MH⁺) = 424. 1H NMR (250 MHz, CDCl₃, free base): δ (ppm): 9.10 (br. s, 1H), 8.99 (dd, 1H), 8.23 (br. d, 1H), 8.24 (br. d, 1H), 8.11 (m, 1H), 7.80 (t, 1H), 7.68 (d, 1H), 7.58 (s, 1H), 7.47 (dd, 1H), 7.15 (m, 1H), 3.87 (br. s, 2H), 3.66 (br. s, 4H), 3.31 (m, 2H), 2.80 (br. s, 4H), 2.40 (s, 3H).

### Example 15

### 2,3-Dihydro-1-(5-methyl-1-phenylpyrazol-4-yl carbonyl)-8-(4-methylpiperazin-1-yl)pyrrolo[3,2-g]isoquinoline (E15)

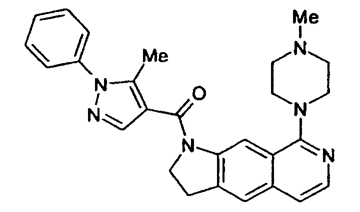

The title compound was prepared from 5-methyl-1-phenylpyrazole-4-carboxylic acid (170 mg, 0.84 mmole) and 2,3-dihydro-8-(4-methylpiperazin-1-yl)pyrrolo[3,2-g]isoquinoline (D13, 150 mg, 0.56 mmole) using a similar procedure to Example 13. The compound was converted to its hydrochloride salt: yellow solid (176 mg, 68%).
MS: m/z (MH⁺) = 453. ¹H NMR (250 MHz, CDCl₃, free base): δ (ppm): 8.62 (br. s, 1H), 8.07 (d, 1H), 7.88 (s, 1H), 7.58 (s, 1H), 7.53-7.46 (m, 5H), 7.16 (d, 1H), 4.38 (t, 2H), 3.45 (br. s, 4H), 3.34 (t, 2H), 2.72 (br. s, 4H), 2.56 (s, 3H), 2.38 (s, 3H).

### Example 16

### 1-(2-Fluorobenzoyl)-2,3-dihydro-8-(4-methylpiperazin-1-yl)pyrrolo(3,2-g]isoquinoline (E16)

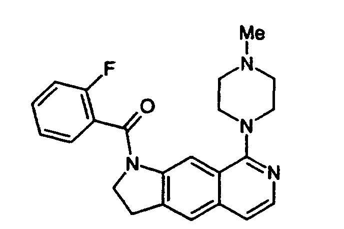

The title compound was prepared from 2-fluorobenzoic acid (157 mg, 1.12 mmole) and 2,3-dibydro-8-(4-methylpiperazin-1-yl)pyrrolo[3,2-g]isoquinoline (D13, 150 mg, 0.56 mmole) using a similar procedure to Example 13. The compound was converted to its hydrochloride salt: yellow solid (176 mg, 74%).
MS: m/z (MH⁺) = 391. 1H NMR (250 MHz, CDCl₃, free base): δ (ppm): 8.97 (s, 1H), 8.08 (d, 1H), 7.58-7.42 (m, 3H), 7.30-7.18 (m, 2H), 7.15 (d, 1H), 4.03 (t, 2H), 3.51 (br. s, 4H), 3.26 (t, 2H), 2.79 (br. s, 4H), 2.41 (s, 3H).

### Example 17

### 2,3-Dihydro-1-(2,4-dimethylthiazol-5-yl carbonyl)-8-(4-methylpiperazin-1-yl)pyrrolo[3,2-g]isoquinoline (E17)

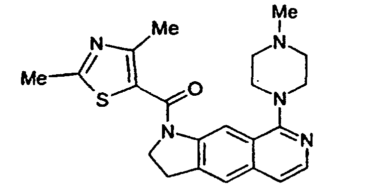

The title compound was prepared from 2,4-dimethylthiazole-5-carboxylic acid (26 mg, 0.17 mmole) and 2,3-dihydro-8-(4-methylpiperazin-1-yl)pyrrolo[3,2-g]isoquinoline (D13, 30 mg, 0.11 mmole) using a similar procedure to Example 13. The compound was converted to its hydrochloride salt: yellow solid (37 mg, 75%).
MS: m/z (MH⁺) = 408. ¹H NMR (250 MHz, CDCl₃, free base): δ (ppm): 8.38 (br. s, 1H), 8.08 (d, 1H), 7.57 (s, 1H), 7.14 (d, 1H), 4.19 (t, 2H), 3.39 (br. s, 4H), 3.31 (t, 2H), 2.74 (s, 3H), 2.67 (br. s, 4H), 2.50 (s, 3H), 2.40 (s, 3H).

### Example 18

### 1-(1-methyl-5-trifluoromethylpyrazol-4-carbonyl)-2,3-dihydro-8-(4-methylpiperazin-1-yl)pyrrolo[3,2-g]isoquinoline (E18)

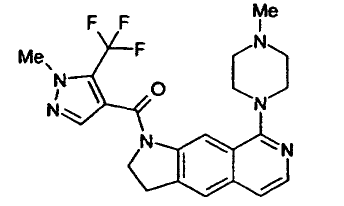

A stirred solution of 2,3-dihydro-8-(4-methylpiperazin-1-yl)pyrrolo[3,2 g]isoquinoline (D13, 107 mg, 0.4 mmole) and ethyl 1-methyl-5-trifluoromethylpyrazole-4-carboxylate (107 mg, 0.48 mmole) in dry toluene (5 ml) under Ar was treated with 2M AlMe₃ in toluene (0.30 ml, 0.6 mmole) and the reaction mixture refluxed for 20h. After cooling, the mixture was transferred to a silica column, eluting with 10% MeOH in CH₂Cl₂. Concentration *in vacuo* afforded a pale beige solid which was transformed into the HCl salt by treatment with 1 M HCl in Et₂O: yellow solid (98 mg, 55%).
MS: m/z (MH) = 445⁺. 1H-NMR (250 MHz, CDCl₃, free base): δ (ppm): 8.85 (br. s, 1H), 8.08 (d, 1H), 7.67 (s, 1H), 7.56 (s, 1H), 7.14 (d, 1H), 4.09 (br. t, 2H), 4.03 (s, 3H), 3.32 (br. s, 4H), 3.29 (t, 2H), 2.70 (br. s, 4H), 2.41 (s, 3H).

### Example 19

### 1-[1-(4-cyanophenyl)-5-ethylpyrazol-4-ylcarbonyl]-2,3-dihydro-8-(4-methylpiperazin-1-yl)pyrrolo[3,2-g]isoquinoline (E19)

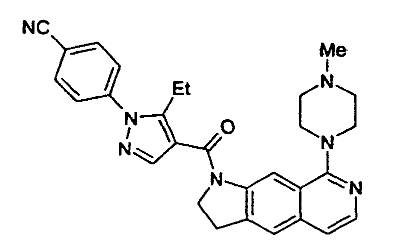

The title compound was prepared from 2,3-dihydro-8-(4-methylpiperazin-1-yl)pyrrolo[3,2-g]isoquinoline (D13, 1.00 g, 3.72 mmole) and ethyl 1-(4-cyanophenyl)-5-ethylpyrazole-4 carboxylate (D16, 1.00 g, 3.72 mmole) according to the procedure described in Example 18: yellow solid (1.36 g, 74%).
MS: m/z (MH) = 492⁺. 1 H-NMR (250 MHz, CDCl₃, free base): δ (ppm): 8.58 (br. s, 1 H), 8.09 (d, 1H), 7.88 (s, 1H), 7.86 (d, 2H), 7.66 (d, 2H), 7.59 (s, 1H), 7.19 (d, 1H), 7.16-7.14 (m, 2H), 4.33 (t, 2H), 3.42 (br. s, 4H), 3.34 (t, 2H), 3.06 (q, 3H), 2.66 (br. s, 4H), 2.36 (s, 3H).

### Example 20

### 1-(2,4-dimethyloxazol-5-ylcarbonyl)-2,3-dihydro-8-(4-methylpiperazin-1-yl)pyrralo[3,2-g]isoquinoline (E20)

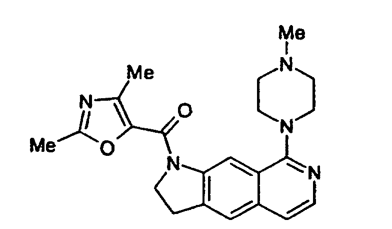

The title compound was prepared from 2,3-dihydro-8-(4-methylpiperazin-1-yl)pyrrolo[3,2-g]isoquinaline (D13, 80 mg, 0.3 mmole) and ethyl 2,4-dimethyloxazole-5-carboxylate *(J. Heterocycl. Chem.* 1998, 35, 859; 85 mg, 0.6 mmole) according to the procedure described in Example 18: yellow solid (60 mg, 51%).
MS: m/z (MH) = 392⁺. 1H NMR (250 MHz, CDCl₃, free base): δ (ppm): 8.75 (br. s, 1H), 8.07 (d, 1H), 7.56 (s, 1H), 7.14 (d, 1H), 4.45 (t, 2H), 3.49 (br. s, 4H), 3.36 (t, 2H), 2.82 (br. s, 4H), 2.52 (s, 6H), 2.46 (s, 3H).

### Example 21

### 2,3-Dihydro-1-(5-methyl-2-trifluoromethylfuran-3-ylcarbonyl)-8-(4-methylpiperazin-1-yl)pyrrolo[3,2-g]isoquinoline (E21)

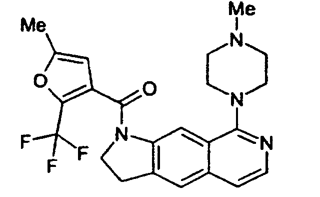

The title compound was prepared from 5-methyl-2-trifluoromethylfuran-3-carboxylic acid (54 mg, 0.28 mmole) and 2,3-dihydro-8-(4-methylpiperazin-1-yl)pyrrolo[3,2-g]isoquinoline (D 13, 50 mg, 0.19 mmole) using a similar procedure to Example 13. The compound was converted to its hydrochloride salt: pale yellow solid (67 mg, 76%).
MS: m/z (MH) = 445⁺. ¹H NMR (250 MHz, CDCl₃, free base): δ (ppm): 8.91 (s, 1H), 8.08 (d, 1H), 7.55 (s, 1H), 7.14 (d, 1H), 6.24 (s, 1H), 4.02 (t, 2H), 3.49 (br. s, 4H), 3.30 (t, 2H), 2.79 (br. s, 4H), 2.42 (s, 6H).

### Example 22

### 1-[1-(4-cyanophenyl)-5-methylpyrazol-4-ylcarbonyl]-2,3-dihydro-8-(4-methylpiperazin-1-yl)pyrrolo[3,2-g]isoquinoline (E22)

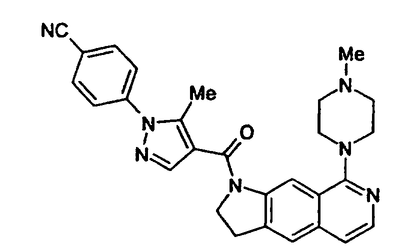

The title compound was prepared from 2,3-dihydro-8-(4-methylpiperazin-1-yl)pyrrolo[3,2-*g*]isoquinoline (D13, 40 mg, 0.15 mmole) and ethyl 1-(4-cyanophenyl)-5-methylpyrazole-4 carboxylate (D 15, 61 mg, 0.24mmole) according to the procedure described in Example 18: yellow solid (31 mg, 42%).
MS: m/z (MH) = 478⁺. 1H-NMR (250 MHz, CDCl₃, free base): δ (ppm): 8.60 (br. s, 1H), 8.07 (d, 1H), 7.90 (s, 1H), 7.82 (d, 2H), 7.66 (d, 2H), 7.56 (s, 1H), 7.23 (d, 1H), 4.33 (t, 2H), 3.44 (br. s, 4H), 3.32 (t, 2H), 2.66 (br. s, 4H), 2.62 (s, 3H), 2.34 (s, 3H).

### Example 23

### 2,3-Dihydro-1-(4-ethyl-2-methyloxazol-5-yl carbonyl)-8-(4-methylpiperazin-1-yl)pyrrolo[3,2-g]isoquinoline (E23)

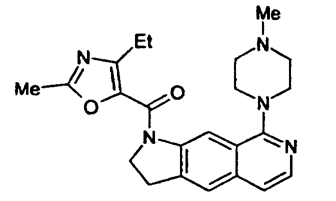

A solution of ethyl 4-ethyl-2-methyloxazole-5-carboxylate (D22 67 mg, 0.36 mmole) and 2,3-dihydro-8-(4-methylpiperazin-1-yl)pyrrolo[3,2-g]isoquinoline (D13, 75 mg, 0.28 mmole) in dry toluene (5 ml), was treated with a solution of AlMe₃ in toluene (2M, 0.2 ml) and stirred, under argon, at 110°C for 20h. The reaction mixture was then cooled and purified directly by flash silica chromatography, eluting with a MeOH/DCM gradient, to afford the title compound as a pale yellow solid. The compound was converted to its hydrochloride salt: yellow solid (52 mg, 42%).
MS: m/z (MH) = 406⁺. ¹H NMR (250 MHz, CDCl₃, free base): δ (ppm): 8.71 (br.s, 1H), 8.07 (d, 1H], 7.56 (s, 1H), 7.14 (d, 1H), 4.43 (t, 2H), 3.47 (br. s, 4H), 3.35 (t, 2H), 2.94 (q, 2H), 2.76 (br. s, 4H), 2.53 (s, 3H), 2.45 (s, 3H), 1.31 (t, 3H).

### Examples E24 - E78

Examples E24 - E78 were prepared according to the procedure described for Examples 13 or 18 using acids or esters from commercial sources except: Examples 54 and 78 (WO 00/35919); Example 61 (*J. Amer. Chem. Soc.* 1992, 114, 8783); Example 65 (Description 30); Example 67 (*Chem. Pharm. Bull.* 1974,22,1814); Example 69 (Description 31); Examples 71-74 (Descriptions 33-36); and Examples 76-77 (Descriptions 37-38).

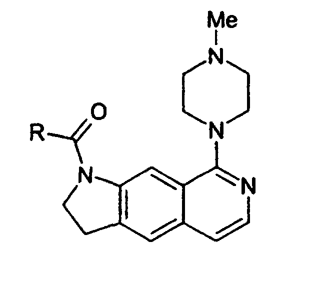

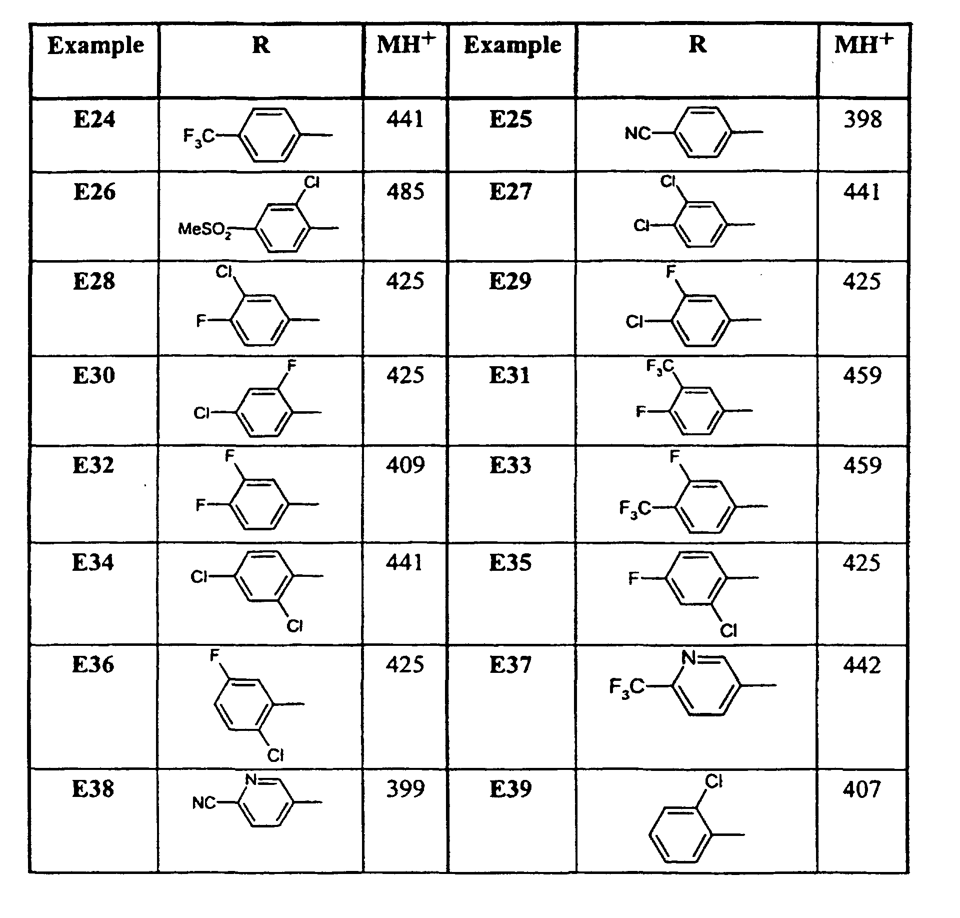

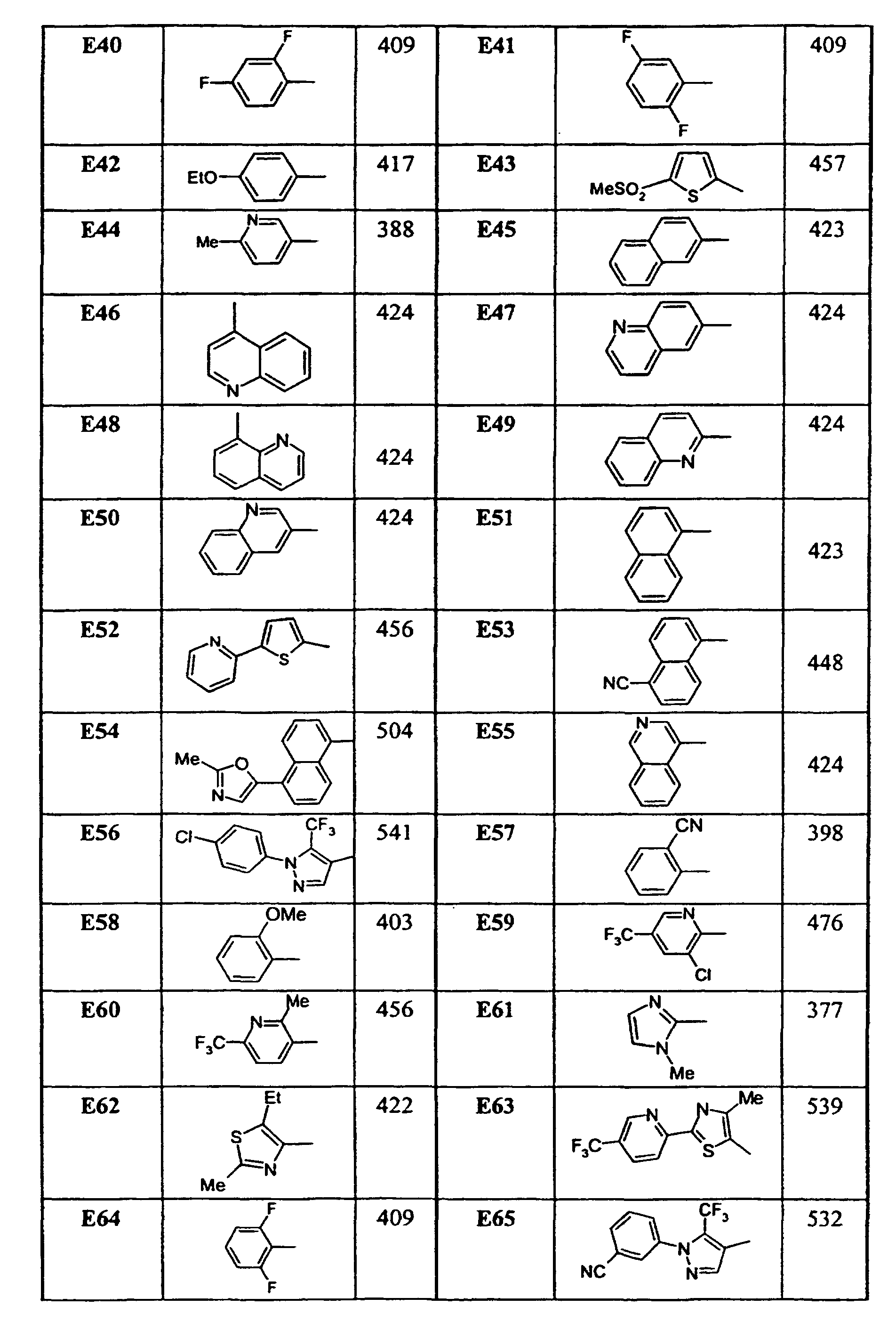

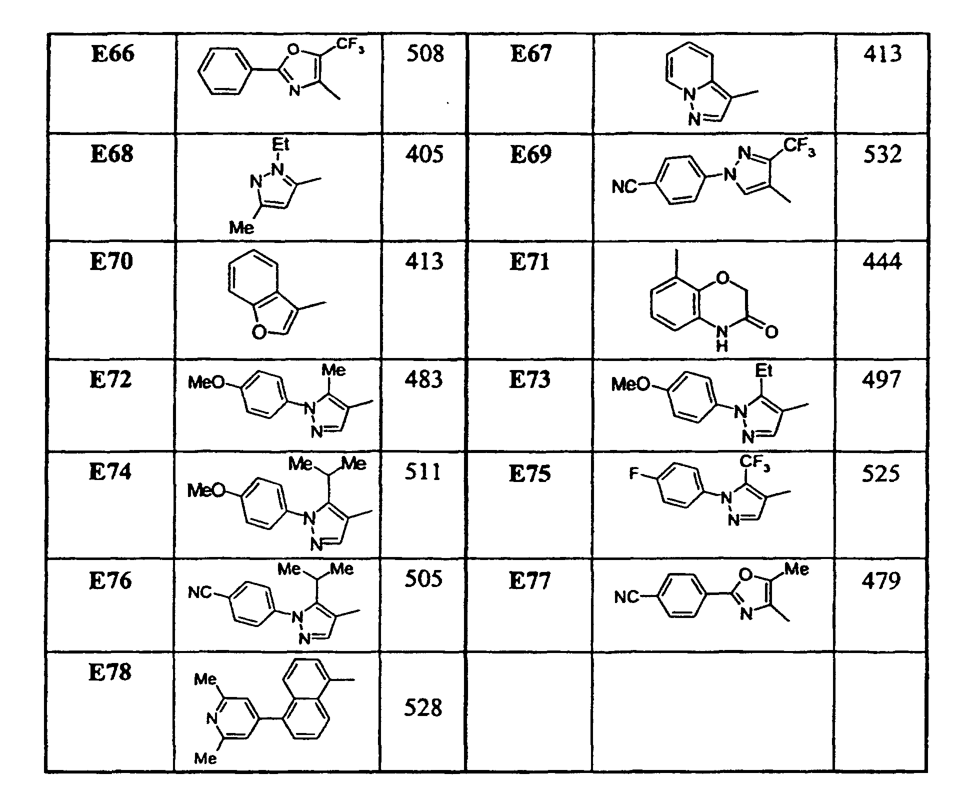

### Example 79

### 1-(2-Chlorophenyl)aminocarbonyl-2,3-dihydro-8(4-methylpiperazin-1-yl)pyrrolo[3,2-g]isoquinoline (E79)

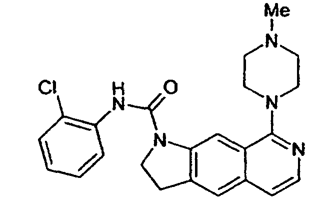

A solution of 2,3-dihydro-8-(4-methylpiperazin-1-yl)pyrrolo[3,2-*g*]isoquinoline (D13, 25 mg, 0.09 mmole) in DCM was treated with 2-chlorophenylisocyanate (16 mg, 0.11 mmole). After 24h the solution was loaded onto an SCX cartridge, washing with DCM and MeOH. The product was eluted with methanolic ammonia and concentrated to dryness, giving a yellow glass, which was converted to the hydrochloride salt by treatment with ethereal HCl (1M) and concentration to dryness: yellow powder (19 mg, 44%).

MS: m/z (MH⁺) = 422/424. ¹H NMR (250 MHz, CDCl₃, free base): δ (ppm): 8.57 (br.s, 1H), 8.39 (m, 1H), 8.05 (d, 1H), 7.53 (s, 1H), 7.38 (m, 2H), 7.07 (d, 1H), 7.04 (m, 2H), 4.24 (t, 2H), 3.42 (m, 6H), 2.74 (br. s, 4H), 2.42 (s, 3H).

**Examples E80 - E84** were prepared using a similar procedure to that described for Example 79.

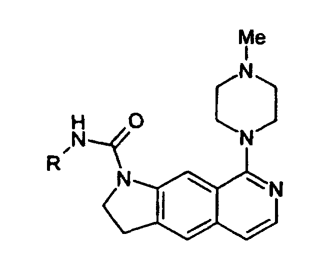

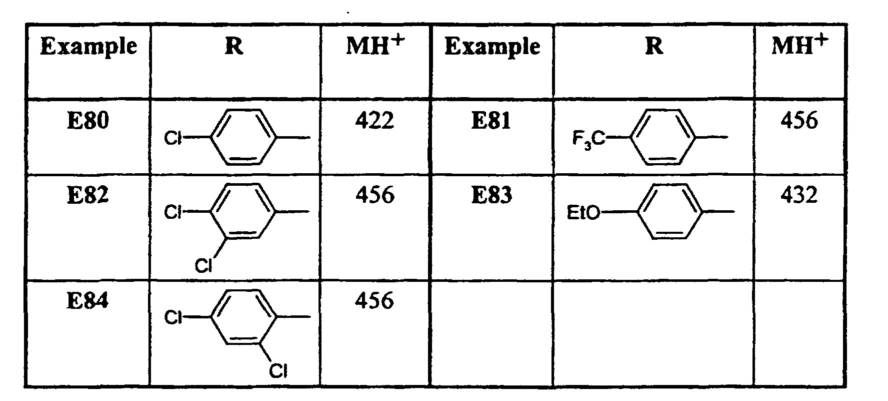

### Example 85

### 1-(2-Fluorobenzoyl)-2,3-dihydro-8-(piperazin-1-yl)pyrrolo[3,2-g]isoquinoline (E85)

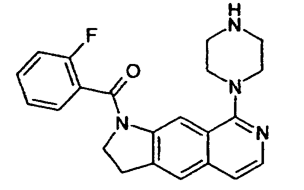

A stirred solution of 2,3-dihydro-8-(4-trifluoroacetylpiperazin-1-yl)pyrrolo[3,2-g]isoquinoline (D21, 49 mg, 0.14 mmole), N,N-diisopropyl carbodiimide(0.036 ml, 0.35 mmole) and HOBT.H₂O (54 mg, 0.35 mmole) in DMF (6 ml) was treated with 2-fluorobenzoic acid (39 mg, 0.28 mmole) and stirred at room temperature for 72h under argon. The reaction was quenched with N-methylamino polystyrene resin (240 mg, -0.35 mmole) and stirred at room temperture for 16h. After filtration of the resin, the solution was transferred to an SCX column, eluting with DCM followed by MeOH. The product was eluted with 1M methanolic ammonia. Concentration *in vacuo* afforded a pale brown solid which was stirred at room temperature for 16h in a solution of MeOH (5 ml) and 1M aqueous K₂CO₃ (0.8 ml). After concentration *in vacuo,* the residue was partitioned between H₂O and CHCl₃. The organic layer was dried over Na₂SO₄ and concentrated *in vacuo.* After purification by preparative TLC using DCM/MeOH (80:20), the product was obtained as a pale beige solid which was transformed into the HCl salt by treatment with 1 M HCl in Et₂O: yellow solid (11 mg, 19%).
MS: m/z (MH⁺) = 377. 1H NMR (250 MHz, CDCl₃, free base): δ (ppm): 8.93 (s, 1H), 8.09 (d, 1H), 7.58 (s, 1H), 7.51 (m, 2H), 7.28 (t, 1H), 7.16 (d, 2H), 4.05 (t, 2H), 3.57 (br. s, 4H), 3.30 (t, 2H), 3.22 (br. s, 4H), 2.94 (br. s, 1H).

### Example 86

### 2,3-Dihydro-8-(1-methylpiperidin-4-yl)pyrrolo[3,2-g]isoquinoline (E86)

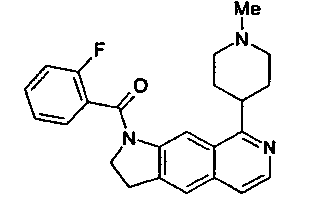

The title compound was prepared from 2,3-dihydro-8-(1-methylpiperidin-4-yl)pyrrolo[3,2-g]isoquinoline (D29, 68 mg, 0.25 mmole) and 2-fluorobenzoic acid according to the procedure in Example 13: yellow powder (61 mg, 56%).
MS: m/z (MH⁺) = 390. 1H NMR (250 MHz, CDCl₃, free base): δ (ppm): 9.11 (s, 1H), 8.41 (d, 1H), 7.62 (s, 1H), 7.57-7.45 (m, 2H), 7.40-7.28 (m, 2H), 7.20 (t, 1H), 4.07 (t, 2H), 3.60 (br. t, 1H), 3.31 (t, 2H), 3.09 (br. d, 2H), 2.38 (s, 3H), 2.37-1.92 (m, 6 H).

### Example 87

### 1-[1-(4-Cyanophenyl)-5-ethylpyrazol-4-ylcarbonyl)-2,3-dihydro-8-(piperazin-1-yl)pyrrolo[3,2-g]isoquinoline (E87)

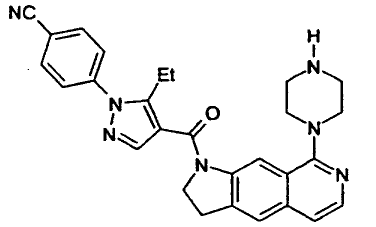

A stirred solution of ethyl 1-(4-cyanophenyl)-5-ethylpyrazole-4-carboxylate (D16, 86 mg, 0.25 mmole) and 2,3-dihydro-8-(4-trifluoroacetylpiperazin-1-yl)pyrrolo[3,2-g]isoquinoline (D21, 95 mg, 0.27 mmol) in dry toluene (10 ml) under Ar was treated with a solution of trimethylaluminium in toluene (2.0 M, 0.24 ml, 0.48 mmol) and heated at 100C for 65 h. After cooling the solution was transferred directly onto a silica gel column, which was eluted with MeOH/DCM (1:4). The filtrate was loaded onto an SCX column, washing with DCM followed by MeOH. The intermediate amide was eluted with methanolic ammonia (1.0 M) in a single fraction, which was concentrated to dryness giving a brown solid. A solution of this residue in MeOH (8 ml) was treated with aqueous K₂CO₃ solution (1.0 M, 2 ml), and stirred for 20 h, then concentrated under vacuum. The residue was diluted with water and extracted with CHCl₃ (4 x). Combined organic phases were dried (Na₂SO₄) and concentrated *in vacuo.* The title compound was obtained by preparative thin layer chromatography on silica gel, eluting with MeOH/DCM (1:4), and converted to the hydrochloride using a solution of HCl in Et₂O (1.0 M) - yellow solid (44 mg, 36%).
MS: m/z (MH⁺) = 478. ¹H NMR (250 MHz, CDCl₃,) δ (ppm): 8.55 (br. s, 1H), 8.08 (d, 1H), 7.88-7.82 (m, 3H), 7.64 (m, 2H), 7.59 (s, 1H), 7.18 (d, 1H), 4.35 (t, 2H), 3.45 (br.s, 4H), 3.35 (t, 2H), 3.20 (br.s, 4H), 3.04 (q, 2H), 1.19 (t, 3H). NH not discernible.

### Example 88

### 2,3-Dibydro-1-(5-methyl-1-phenylpyrazolo-yl carbonyl)-8-(piperazin-1-yl)pyrrolo[3,2-g]isoquinoline (E88)

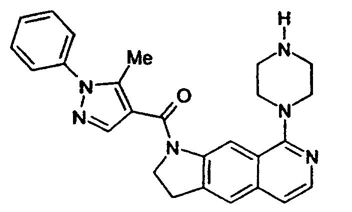

The title compound was prepared from ethyl 5-methyl-1-phenylpyrazole-4-carboxylate (76 mg, 0.33 mmole) and 2,3-dihydro-8-(4-trifluoroacetylpiperazin-1-yl)pyrrolo[3,2-g]isoquinoline (D21, 95 mg, 0.27 mmol) using a similar procedure to Example 83. The compound was converted to its hydrochloride salt: yellow solid (74 mg, 48%).
MS: m/z (MH⁺) = 439. ¹H NMR (250 MHz, CDCl₃, free base): δ (ppm): 8.59 (br. s, 1H), 8.06 (d, 1H), 7.87 (s, 1H), 7.58-7.40 (m, 6H), 7.13 (d, 1H), 4.36 (t, 2H), 3.35 (br. s, 4H), 3.30 (t, 2H), 3.10 (br. s, 4H), 2.53 (s, 3H). NH not discernible.

### Example 89

### 1-(2-Chlorophenyl-1-aminocarbonyl)-8-(piperazin-1-yl)-2,3-dihydropyrrolo [3,2-g]isoquinoline (E89)

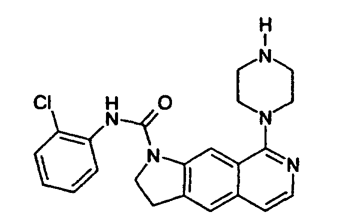

A stirred solution of 2,3-dihydro-8-(4-trifluoroacetylpiperazin-1-yl)pyrrolo[3,2-g]isoquinoline (D21, 84 mg, 0.24 mmole) in DCM (5 ml) was treated with 2-chlorophenylisocyanate (74 mg, 0.48 mmole). After 65 h the solution was loaded onto an SCX column, washing with DCM followed by MeOH. The intermediate urea was eluted with methanolic ammonia (1.0 M) in a single fraction, which was concentrated to dryness giving a brown solid. A solution of this residue in MeOH (8 ml) was treated with aqueous K₂CO₃ solution (1.0 M, 2 ml), and stirred for 20 h, then concentrated under vacuum. The residue was diluted with water and extracted with CHCl₃ (4 x). Combined organic phases were dried (Na₂SO₄) and concentrated *in vacuo.* The title compound was obtained by preparative thin layer chromatography on silica gel, eluting with MeOH/DCM (1:4), and converted to the hydrochloride using a solution of HCl in Et₂O (1.0 M) - yellow solid (40 mg, 38%).
MS: m/z (MH⁺) = 408. ¹H NMR (250 MHz, CDCl₃) δ (ppm): 8.58 (s, 1H), 8.38 (dd, 1H), 8.05 (d, 1H), 7.52 (s, 1H), 7.40-7.30 (m, 2H), 7.13 (d, 1H), 7.03 (dt, 1H), 4.23 (t, 2H), 3.43-3.36 (m, 6H), 3.19-3.16 (m, 4H). Piperazine NH and urea NH not discernible.

## Claims

1. A compound of formula (1) or a pharmaceutically acceptable salt thereof: 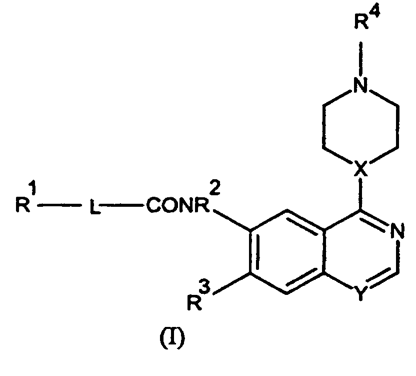 in which R¹ is selected from a group of formula (i) or (ii) 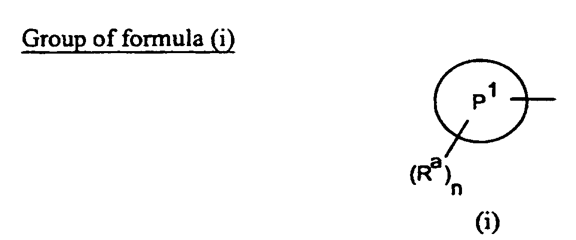 where P¹ is phenyl, naphthyl, a 5 or 6 membered heteroaryl ring containing 1 to 3 heteroatoms selected from oxygen, nitrogen and sulphur, a benzofused heterocyclic ring containing 1 to 3 heteroatoms selected from oxygen, nitrogen and sulphur, or a pyridofused heterocyclic ring containing 1 to 3 nitrogen atoms;
R^{a} is halogen, C₁₋₆alkyl, C₃₋₆cycloalkyl, CF₃, C₁₋₆alkoxy, OCF₃, hydroxy, cyano, nitro, hydroxyC₁₋₆alkyl, COC₁₋₆alkyl, CO₂R⁵, SO₂R⁵, NR⁵R⁶, CONR⁵R⁶, and SO₂NR⁵R⁶ where R⁵ and R⁶ are independently hydrogen or C₁₋₆alkyl;
n is 0, 1, 2 or 3; 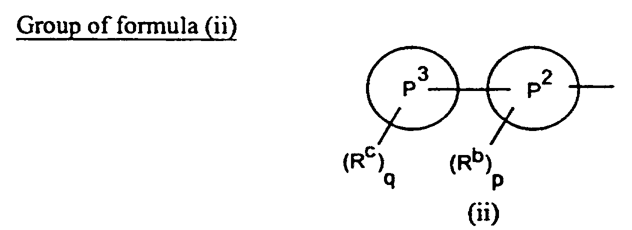 in which P² and P³ are independently as defined for P¹ above;
R^{b} and R^{c} are independently as defined for R^{a} above;
p and q are independently as defined for n above;
L is a single bond or NH;
R² is hydrogen or together with the group R³ forms a further group -CH = CH- or -(CR⁷R⁸)₂- where R⁷ and R⁸ are independently hydrogen or C₁₋₆alkyl;
R³ is hydrogen or together with R² forms a further group as defined above;
Y is N or CH;
XisNorCH;
R⁴ is hydrogen or C₁₋₆alkyl.

2. A compound according to claim 1 in which Y is CH.

3. A compound according to claim 1 or 2 in which X is N.

4. A compound according to any of the preceding claims in which R² is hydrogen or together with R³ forms a further group -(CH₂)₂-.

5. A compound according to any the preceding claims in which R⁴ is a methyl group.

6. A compound according to any of the preceding claims in which R¹ is a group of formula (i) wherein P¹ is phenyl or oxazolyl.

7. A compound according to any one of claims 1 - 5 in which R¹ is a group of formula (ii) wherein P² is pyrazolyl and P³ is phenyl.

8. A compound according to claim 1 which is:
2,3-Dihydro-1-(5-methyl-1-phenylpyrazol-4-yl carbonyl)-8-(4-methylpiperazin-1-yl)pyrrolo[3,2-g]isoquinoline,
1-(2-Fluorobenzoyl)-2,3-dihydro-8-(4-methylpiperazin-1-yl)pyrrolo[3,2-g]isoqinoline,
1-[1-(4-cyanophenyl)-5-ethylpyrazol-4-ylcarbonyl]-2,3-dihydro-8-(4-methylpiperazin-1-yl)pyrrolo[3,2-g]isoquinoline,
1-[1-(4-cyanophenyl)-5-methylpyrazol-4-ylcarbonyl]-2,3-dihydro-8-(4-methylpiperazin-1-yl)pyrrolo[3,2-g]isoquinoline,
2,3-Dihydro-1-(4-ethyl-2-methyloxazol-5-ylcarbonyl)-8-(4-methylpiperazin-1-yl)pyrrolo[3,2-g]isoquinoline
or a pharmaceutically acceptable salt thereof.

9. A process for the preparation of a compound of formula (I) according to claim 1 or a pharmaceutically acceptable salt which comprises either:
(a) when L is single bond, coupling a compound of formula (II)
R¹―A (II)
in which R¹ is as defined in formula (I) and A is an activated carboxylic acid group with a compound of formula (III); 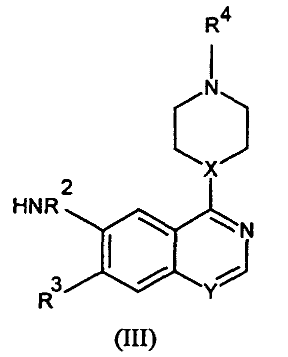 in which R², R³, R⁴, X and Y are as defined in formula (I); or
(b) where L is NH, coupling a compound of formula (IV)
R¹―B (IV)
in which R¹ is as defined in formula (I) and B is either -N=C=O or is NH₂ in the presence an appropriate urea forming agent, with a compound of formula (III) as defined above;
and optionally thereafter for either process (a) or (b):
• removing any protecting groups;
• forming a pharmaceutically acceptable salt.

10. A compound according to any of claims 1 to 8 for use in therapy.

11. A compound according to any of claims 1 to 8 for use in treatment of depression.

12. A pharmaceutical composition which comprises a compound according to any of claims 1 to 8 or a pharmaceutically acceptable salt thereof and a pharmaceutically acceptable carrier or excipient.

13. The use of a compound according to any one of claims 1 to 8 or a pharmaceutically acceptable salt thereof, in the manufacture of a medicament for the treatment or prophylaxis of diseases or disorders in which a ligand with affinity for 5-HT_{1A}, 5-HT_{1B} and 5-HT_{1D} receptors is beneficial.

## Patentansprüche

1. Verbindung der Formel (I) oder ein pharmazeutisch verträgliches Salz davon: 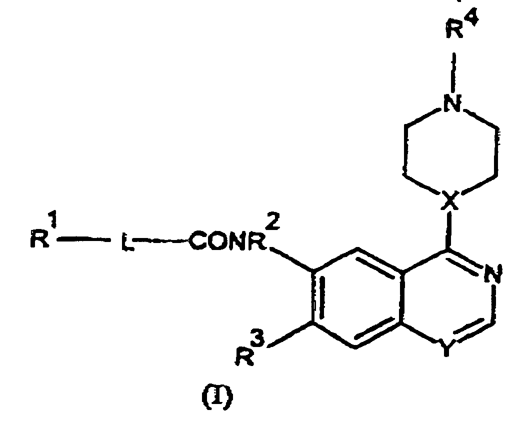 wobei der Rest R¹ aus einer Gruppe der Formel (i) oder (ii) ausgewählt ist 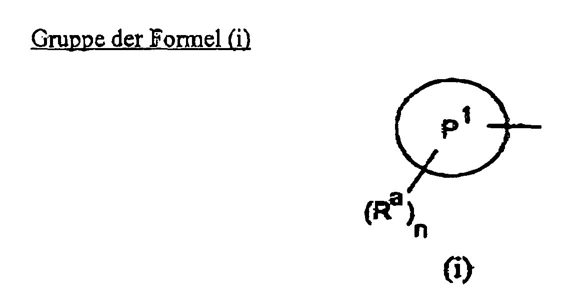 wobei P¹ Phenyl, Naphtyl, ein 5- oder 6-gliedriger Heteroarylring, welcher 1 bis 3 Heteroatome enthält, die aus Sauerstoff, Stickstoff und Schwefel ausgewählt sind, ein benzokondensierter heterocyclischer Ring, welcher 1 bis 3 Heteroatome enthält, die aus Sauerstoff, Stickstoff und Schwefel ausgewählt sind, oder ein pyridokondensierter heterocyclischer Ring, welcher 1 bis 3 Stickstoffatome enthält, ist;
der Rest R^{a} Halogen, C₁₋₆-Alkyl, C₃₋₆-Cycloalkyl, CF₃, C₁₋₆-Alkoxy, OCF₃, Hydroxy, Cyano, Nitro, Hydroxy-C₁₋₆-Alkyl, COC₁₋₆-Alkyl, CO₂R⁵, SO₂R⁵, NR⁵R⁶, CONR⁵R⁶, und SO₂NR⁵R⁶ ist, wobei die Reste R⁵ und R⁶ unabhängig Wasserstoff oder C₁₋₆-Alkyl sind;
n gleich 0, 1, 2 oder 3 ist; 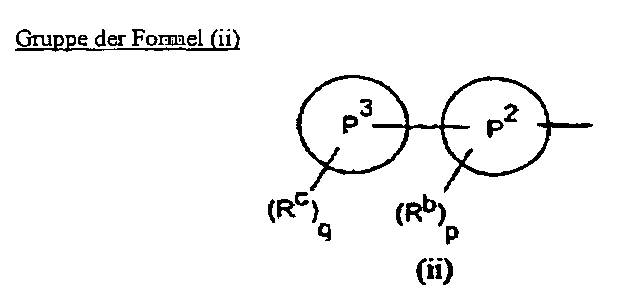 wobei P² und P³ unabhängig wie für P¹ vorstehend definiert sind;
die Reste R^{b} und R^{c} unabhängig wie für den Rest R^{a} vorstehend definiert sind;
p und q unabhängig wie für n vorstehend definiert sind;
L eine Einfachbindung oder NH ist;
der Rest R² Wasserstoff ist oder zusammen mit dem Rest R³ eine weitere Gruppe ―CH=CH― oder (CR⁷R⁸)₂― bildet, wobei die Reste R⁷ und R⁸ unabhängig Wasserstoff oder C₁₋₆-Alkyl sind;
der Rest R³ Wasserstoff ist oder zusammen mit dem Rest R² eine weitere Gruppe, wie vorstehend definiert, bildet;
Y die Bedeutung N oder CH hat;
X die Bedeutung N oder CH hat;
der Rest R⁴ Wasserstoff oder C₁₋₆-Alkyl ist.

2. Verbindung gemäß Anspruch 1, wobei Y die Bedeutung CH hat.

3. Verbindung gemäß Anspruch 1 oder 2, wobei X die Bedeutung N hat.

4. Verbindung gemäß einem der vorhergehenden Ansprüche, wobei der Rest R² Wasserstoff ist oder zusammen mit dem Rest R³ eine weitere Gruppe ―(CH₂)₂― bildet.

5. Verbindung gemäß einem der vorhergehenden Ansprüche, wobei der Rest R⁴ eine Methylgruppe ist.

6. Verbindung gemäß einem der vorhergehenden Ansprüche, wobei der Rest R¹ eine Gruppe der Formel (i) ist, wobei P¹ Phenyl oder Oxazolyl ist.

7. Verbindung gemäß einem der Ansprüche 1 - 5, wobei der Rest R' eine Gruppe der Formel (ii) ist, wobei P² Pyrazolyl ist und P³ Phenyl ist.

8. Verbindung gemäß Anspruch 1, welche:
2,3-Dihydro-1-(5-methyl-1-phenylpyrazol-4-ylcarbonyl)-8-(4-methylpiperazin-1-yl)pyrrolo[3,2-g]isochinolin,
1-(2-Fluorbenzoyl)-2,3-dihydro-8-(4-methylpiperazin-1-yl)pyrrolo[3,2-g]isochinolin,
1-[1-(4-Cyanophenyl)-5-ethylpyrazol-4-ylcarbonyl]-2,3-dihydro-8-(4-methylpiperazin-1-yl)pyrrolo[3,2-g]isochinolin,
1-[1-(4-Cyanophenyl)-5-methylpyrazol-4-ylcarbonyl]-2,3-dihydro-8-(4-methylpiperazin-1-yl)pyrrolo[3,2-g]isochinolin,
2,3-Dihydro-1-(4-ethyl-2-methyloxazol-5-ylcarbonyl)-8-(4-methy)piperazin-1-yl)pyrrolo[3,2-g]isochinolin
oder ein pharmazeutisch verträgliches Salz davon ist.

9. Verfahren zur Herstellung einer Verbindung der Formel (1) gemäß Anspruch 1 oder eines pharmazeutisch verträglichen Salzes, welches entweder:
(a) wenn L eine Einfachbindung ist, das Kuppeln einer Verbindung der Formel (II)
R¹―A (II)
wobei der Rest R¹ wie in Formel (I) definiert ist und A eine aktivierte Carbonsäuregruppe ist, mit einer Verbindung der Formel (III) umfaßt; 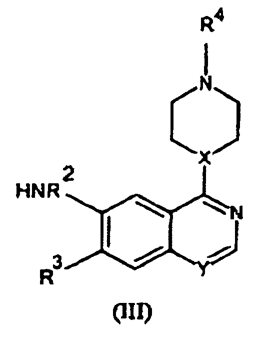 wobei die Reste R², R³, R⁴, X und Y wie in Formel (I) definiert sind; oder
(b) wenn L die Bedeutung NH hat, Kuppeln einer Verbindung der Formel (IV)
R¹―B (IV)
wobei der Rest R¹ wie in Formel (I) definiert ist und B entweder -N=C=O ist oder NH₂ ist, in Gegenwart eines geeigneten Harnstoff-bildenden Mittels, mit einer Verbindung der Formel (III), umfaßt, welche wie vorstehend definiert ist;
und gegebenenfalls danach für beide Verfahren (a) oder (b):
- Entfernen von allen Schutzgruppen;
- Bilden eines pharmazeutisch verträglichen Salzes.

10. Verbindung gemäß einem der Ansprüche 1 bis 8 zur Verwendung in der Therapie.

11. Verbindung gemäß einem der Ansprüche 1 bis 8 zur Verwendung in der Behandlung von Depressionen.

12. Arzneimittel, welches eine Verbindung gemäß einem der Ansprüche 1 bis 8 oder ein pharmazeutisch verträgliches Salz davon und einen pharmazeutisch verträglichen Träger oder Exzipienten umfasst.

13. Verwendung einer Verbindung gemäß einem der Ansprüche 1 bis 8 oder eines pharmazeutisch verträglichen Salzes davon zur Herstellung eines Medikaments zur Behandlung oder Prophylaxe von Krankheiten oder Störungen, bei denen ein Ligand mit einer Affinität zu 5-HT_{1A}-, 5-HT_{1B}- und 5-HT_{1D}-Rezeptoren vorteilhaft ist.

## Revendications

1. Composé de formule (I) ou un de ses sels pharmaceutiquement acceptables : 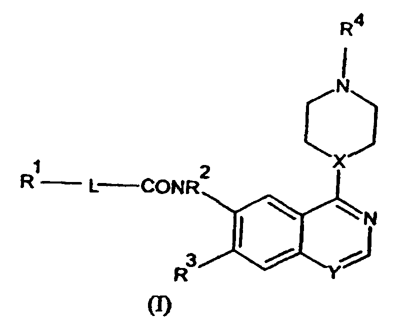 dans laquelle R¹ est choisi entre un groupe de formule (i) et un groupe de formule (ii) 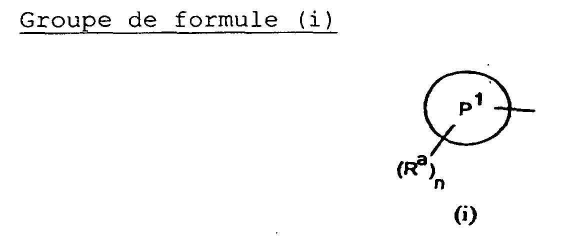 dans laquelle P¹ représente un groupe phényle, un groupe naphtyle, un noyau hétéroaryle penta- ou hexagonal contenant 1 à 3 hétéroatomes choisis entre l'oxygène, l'azote et le soufre, un noyau hétérocyclique benzo-condensé contenant 1 à 3 hétéroatomes choisis entre l'oxygène, l'azote et le soufre, ou un noyau hétérocyclique pyrido-condensé contenant 1 à 3 atomes d'azote ;
R^{a} représente un groupe halogéno, alkyle en C₁ à C₆, cycloalkyle en C₃ à C₆, CF₃, alkoxy en C₁ à C₆, OCF₃, hydroxy, cyano, nitro, hydroxyalkyle en C₁ à C₆, CO(alkyle en C₁ à C₆), CO₂R⁵, SO₂R⁵, NR⁵R⁶, CONR⁵R⁶ ou SO₂NR⁵R⁶ dans lequel R⁵ et R⁶ représentent indépendamment un atome d'hydrogène ou un groupe alkyle en C₁ à C₆ ;
n est égal à 0, 1, 2 ou 3 ; 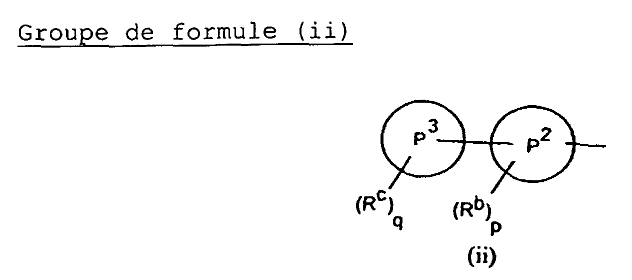 dans laquelle P² et P³ répondent indépendamment à la définition mentionnée pour P¹ ci-dessus ;
R^{b} et R^{c} répondent indépendamment à la définition pour R^{a} ci-dessus ;
p et q répondent indépendamment à la définition mentionnée pour n ci-dessus ;
L représente une liaison simple ou un groupe NH ;
R² représente un atome d'hydrogène ou, conjointement avec le groupe R³, forme un groupe supplémentaire -CH=CH- ou (CR⁷R⁸)₂- dans lequel R⁷ et R⁸ représentent indépendamment un atome d'hydrogène ou un groupe alkyle en C₁ à C₆ ;
R³ représente un atome d'hydrogène ou, conjointement avec R², forme un groupe supplémentaire répondant à la définition précitée ;
Y représente un atome de N ou un groupe CH ;
X représente un atome de N ou un groupe CH ;
R⁴ représente un atome d'hydrogène ou un groupe alkyle en C₁ à C₆.

2. Composé suivant la revendication 1, dans lequel Y représente un groupe CH.

3. Composé suivant la revendication 1 ou 2, dans lequel X représente un atome de N.

4. Composé suivant l'une quelconque des revendications précédentes, dans lequel R² représente un atome d'hydrogène ou, conjointement avec R³, forme un groupe supplémentaire -(CH₂)₂-.

5. Composé suivant l'une quelconque des revendications précédentes, dans lequel R⁴ représente un groupe méthyle.

6. Composé suivant l'une quelconque des revendications précédentes, dans lequel R¹ représente un groupe de formule (i) dans laquelle P¹ représente un groupe phényle ou oxazolyle.

7. Composé suivant l'une quelconque des revendications 1 à 5, dans lequel R¹ représente un groupe de formule (ii) dans laquelle P² représente un groupe pyrazolyle et P³ représente un groupe phényle.

8. Composé suivant la revendication 1, qui est :
la 2,3-dihydro-1-(5-méthyl-1-phénylpyrazol-4-yl carbonyl)-8-(4-méthylpipérazine-1-yl)pyrrolo[3,2-g]isoquinoléine,
la 1-(2-fluorobenzoyl)-2,3-dihydro-8-(4-méthylpipérazine-1-yl) pyrrolo [3,2-g]isoquinoléine,
la 1-[1-(4-cyanophényl)-5-éthylpyrazol-4-ylcarbonyl]-2,3-dihydro-8-(4-méthylpipérazine-1-yl)pyrrolo[3,2-g]isoquinoléine,
la 1-[1-(9-cyanophényl)-5-méthylpyrazol-4-ylcarbonyl]-2,3-dihydro-8-(4-méthylpipérazine-1-yl)pyrrolo[3,2-g]isoquinoléine,
la 2,3-dihydro-l-(4-éthyl-2-méthyloxazol-5-ylcarbonyl)-8-(4-méthylpipérazine-1-yl)pyrrolo[3,2-g]isoquinoléine,
ou un de ses sels pharmaceutiquement acceptables.

9. Procédé pour la préparation d'un composé de formule (I) suivant la revendication 1 ou d'un sel pharmaceutiquement acceptable, qui comprend soit :
(a) lorsque L représente une liaison simple, le couplage d'un composé de formule (II)
R¹―A (II)
dans laquelle R¹ répond à la définition mentionnée pour la formule (I) et A représente un groupe acide carboxylique activé, avec un composé de formule (III) ; 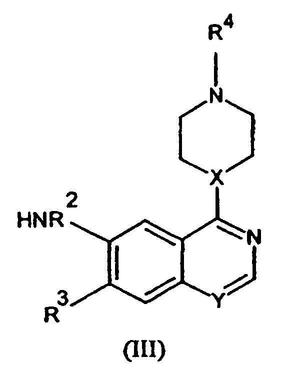 dans laquelle R², R³, R⁴, X et Y répondent aux définitions mentionnées pour la formule (I) ; soit
(b) lorsque L représente un groupe NH, le couplage d'un composé de formule (IV)
R¹―B (IV)
dans laquelle R¹ répond à la définition mentionnée pour la formule (I) et B représente un groupe -N=C=O ou représente un groupe NH² en présence d'un agent approprié de formation d'urée, avec un composé de formule (III) répondant à la définition précitée ;
et ensuite, facultativement, pour le procédé (a) ou (b) :
• l'élimination de n'importe quels groupes protecteurs ;
• la formation d'un sel pharmaceutiquement acceptable.

10. Composé suivant l'une quelconque des revendications 1 à 8, destiné à être utilisé en thérapie.

11. Composé suivant l'une quelconque des revendications 1 à 8, destiné à être utilisé dans le traitement de la dépression.

12. Composition pharmaceutique qui comprend un composé suivant l'une quelconque des revendications 1 à 8 ou un de ses sels pharmaceutiquement acceptables et un support ou excipient pharmaceutiquement acceptable.

13. Utilisation d'un composé suivant l'une quelconque des revendications 1 à 8 ou d'un de ses sels pharmaceutiquement acceptables dans la production d'un médicament destiné au traitement ou à la prophylaxie de maladies ou d'affections dans lesquelles un ligand présentant une affinité pour les récepteurs 5-HT_{1A}, 5-HT_{1B} et 5-HT_{1D} est bénéfique.
